# EUROPEAN PATENT SPECIFICATION

(11) **EP 1 604 987 B1**
(45) Date of publication and mention of the grant of the patent: **21.05.2008**
(21) Application number: 05015518.3
(22) Date of filing: 26.06.2002
(51) Int. Cl.: C07D 321/00, C08G 63/85

(54) **Shaping of macrocyclic oligoesters**
Formgebende Verarbeitung von makrozyklischen Oligoestern
Mise en forme d'oligoesters macrocycliques

(30) Priority: 27.06.2001 US 301399 P
(43) Date of publication of application: 14.12.2005
(62) Divisional of application: 02756358.4
(73) Proprietor: Cyclics Corporation, Schenectady, NY 12308 (US)
(72) Inventor: Thompson, Timothy A. c/o Cyclics Corporation, Schenectady, NY 12308 (US); Phelps, Peter D., Schenectady, NY 12306 (US); Winckler, Steven J., Troy, NY 12180 (US)
(74) Representative: W.P. Thompson & Co.

(56) References cited:
- WO-A-02/18476
- CH-A- 654 304
- FR-A- 2 530 628
- GB-A- 2 123 405
- JP-A- 57 122 078
- SU-A- 1 532 560
- US-A- 4 803 288

## Description

### Related Application

This application claims the benefit of the filing date of U.S. Provisional Application Serial No. 60/301,399, filed on June 27, 2001, entitled "Melt Isolation, Solidification, and Formulation of Macrocyclic Oligoesters," which is incorporated by reference herein in its entirety.

### Technical Field

The invention relates generally to thermoplastics and articles formed therefrom. More particularly, the invention relates to processes for isolating, formulating, and shaping macrocyclic oligoesters such as macrocyclic oligoesters of 1,4-butylene terephthalate.

### Background Information

Linear polyesters such as poly(alkylene terephthalate) are generally known and commercially available where the alkylene typically has 2 to 8 carbon atoms. Linear polyesters have many valuable characteristics including strength, toughness, high gloss, and solvent resistance. Linear polyesters are conventionally prepared by the reaction of a diol with a dicarboxylic acid or its functional derivative, typically a diacid halide or ester. Linear polyesters may be fabricated into articles of manufacture by a number of known techniques including extrusion, compression molding, and injection molding.

Recently, macrocyclic oligoesters were developed that have unique properties that make them attractive for a variety of applications, including as matrices for engineering thermoplastic composites. Macrocyclic oligoesters exhibit low melt viscosity, for example, allowing them easily to impregnate a dense fibrous preform followed by polymerization to polymers. Furthermore, certain macrocyclic oligoesters melt and polymerize at temperatures well below the melting point of the resulting polymer. Upon melting and in the presence of an appropriate catalyst, polymerization and crystallization can occur virtually isothermally.

Production of macrocyclic oligoesters such as macrocyclic (1,4-butylene terephthalate) typically involves the use of one or more solvents such as o-dichlorobenzene or xylene. Some prior techniques that have been used to recover macrocyclic oligoesters dissolved in a solvent required the addition of a large amount of anti-solvent to the solution to precipitate the macrocyclic oligoester followed by collection of the product using a filter or a centrifuge. The use of anti-solvents results in increased processing complexity, costs, and creates additional environmental storage and disposal concerns.

Linear polyesters may be depolymerized to form macrocyclic oligoesters. The product solution of a depolymerization reaction may be dilute, making recovery more time consuming. Depolymerization production efforts also generally take place in stages, with each stage including a step of the process and with intermediate storage between the steps.

**[0006a]** International Publication Number WO02/18476 describes a method for preparing macrocyclic oligoester compositions from intermediate molecular weight polyesters.

**[0006b]** Russian Patent Publication SU 1532560 describes various polyether structures.

**[0006c]** U.S. Patent No. 4,803,288 describes a process for producing a Macrocyclic ester compound by decomposing and cyclizing linear ester compounds with the addition of a glycol or oligoester compound.

**[0006d]** Swiss Patent Publication No. CH 654304, UK Patent Application Publication No. 2123405, French Patent Publication No. 2530628, and Japanese Patent Publication No. 57-122078 also describe a process for producing a macrocyclic ester by decomposing and cyclizing linear ester compounds with the addition of a glycol or oligoester compound.

### Summary of the Invention

There is a need for effective, efficient, and low cost processes for isolating, formulating, and shaping macrocyclic oligoesters. There is also a need for processes that enable continuous production of macrocyclic oligoesters. In one aspect, the invention generally relates to processes for producing macrocyclic oligoesters (e.g., macrocyclic 1,4-butylene terephthalate oligomers), including processes for isolating macrocyclic oligoesters from solvents so the resulting macrocyclic oligoesters are substantially free from solvent. The invention also includes processes for formulating and shaping the substantially solvent free macrocyclic oligoesters. In some embodiments, the described processes are performed continuously, to enable continuous production in a manufacturing plant. Further, the described processes can be beneficially combined for greater efficiencies and production benefits.

In one aspect, the invention features a process for isolating a macrocyclic oligoester. A solution including a macrocyclic oligoester and a solvent is provided. The macrocyclic oligoester typically has a structural repeat unit of formula (I): 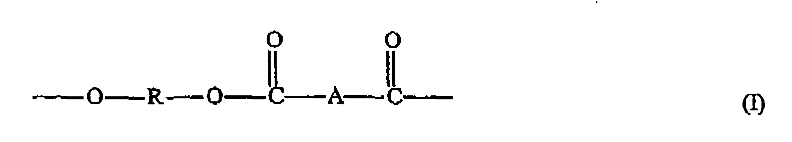 where R may be an alkylene, a cycloalkylene, or a mono- or polyoxyalkylene group, and A may be a divalent aromatic or alicyclic group. The solvent is then removed without the use of anti-solvent. In typical practice, substantially all of the solvent is removed. In one embodiment, the solvent is removed under elevated temperature conditions. In another embodiment, the solvent is removed under reduced pressure conditions. In another embodiment, the solvent is removed under a combination of both elevated temperature and reduced pressure conditions. The macrocyclic oligoester, which is substantially free from the solvent then typically is collected. In one embodiment, the solvent is continuously removed from the solution. In another embodiment, the macrocyclic oligoester substantially free from the solvent is continuously collected.

In another aspect, the invention features a process for shaping a partially-crystallized macrocyclic oligoester. In one embodiment, this process includes providing a substantially solvent-free molten macrocyclic oligoester typically having the structural repeat unit of the formula (I) described above. The substantially solvent-free molten macrocyclic oligoester is sheared to form a partially-crystallized macrocyclic oligoester. The partially-crystallized macrocyclic oligoester is then shaped. In one embodiment, a continuous flow of substantially solvent-free molten macrocyclic oligoester is sheared continuously. In another embodiment, the step of shaping the partially-crystallized macrocyclic oligoester is conducted continuously.

In yet another aspect, the invention features a process for malcing a prepreg of a macrocyclic oligoester and a polymerization catalyst. In one embodiment, a mixture of a molten macrocyclic oligoester and a polymerization catalyst, which is substantially free from any solvent, is provided. The macrocyclic oligoester typically has the structural repeat unit of the formula (I) described above. The mixture is deposited onto a fabric material, forming a prepreg. In one embodiment, the mixture is partially-crystallized prior to being deposited onto the fabric material.

In yet another aspect, the invention features a process for making a prepreg of a macrocyclic oligoester and a polymerization catalyst. In one embodiment, a mixture of a molten macrocyclic oligoester and a polymerization catalyst, which is substantially free from any solvent, is provided continuously. The macrocyclic oligoester typically has the structural repeat unit of formula (I) described above. The mixture of the macrocyclic oligoester and the polymerization catalyst is crystallized partially and deposited onto a fabric material.

In still another aspect, the invention features a process for formulating a macrocyclic oligoester. In one embodiment, a solution including a macrocyclic oligoester and a solvent is provided. The macrocyclic oligoester typically has a structural repeat unit of formula (I) described above. The solvent often is continuously removed from the solution at a temperature between about 180°C and about 200°C and at a pressure between about atmospheric pressure and about 10 torr. The step of solvent removal produces a substantially solvent-free molten macrocyclic oligoester. The substantially solvent-free molten macrocyclic oligoester then is sheared at a temperature below the melting point of the molten macrocyclic oligoester. In one embodiment, the shearing temperature is maintained between about 145°C and about 155°C, thereby forming a partially-crystallized macrocyclic oligoester. The partially-crystallized macrocyclic oligoester is shaped into one or more shapes such as a pellet, a pastille, and/or a flake.

### Brief Description of Figures

FIG. 1 is a schematic flow diagram of an embodiment of a solvent removal system.

FIG. 2 is a schematic flow diagram of an embodiment of a solvent removal system.

FIG. 3 is a schematic flow diagram of an embodiment of a solvent removal system.

FIG. 4 is a schematic flow diagram of an embodiment of a solvent removal system.

FIG. 5 is a schematic flow diagram of an embodiment of a process for making pellets from a macrocyclic oligoester.

FIG. 6 is a schematic flow diagram of an embodiment of a pastillation process (e.g., making prepregs from a macrocyclic oligoester).

FIG. 7 is a schematic illustration of an embodiment of a process for making a prepreg from a macrocyclic oligoester.

FIG. 8 is a schematic flow diagram of an embodiment of a solvent removal system.

FIG. 9 is a schematic flow diagram of an embodiment of a system for shaping macrocyclic oligoesters from a solution of macrocyclic oligoester and solvent.

FIG. 10 is a schematic flow diagram of an embodiment of a system for shaping macrocyclic oligoesters from a solution of macrocyclic oligoester and solvent.

### Description

The processes of the invention are more efficient and economical than existing techniques because the isolation, formulation, and shaping processes may be carried out continuously and on a large scale. The purity of the macrocyclic oligoester may be effectively controlled by the incorporation of multiple solvent removal apparatus where necessary. The isolation, formulation, and shaping processes also may be beneficially linked, resulting in efficient mass production and lowered manufacturing costs. Such linked processes avoid product and energy waste incurred when the isolation, formulation, and shaping processes are conducted separately. For example, macrocyclic oligoesters may be isolated in a molten state. The shaping process typically requires the macrocyclic oligoesters to be provided in a molten state. Accordingly, linking these processes reduces energy uses and increases production efficiency.

For example of the benefits of continuous production, a macrocyclic oligoester having between about 80 ppm and about 400 ppm solvent may be produced at a rate of between about 40 kg/hr and about 300 kg/hr using a feed solution having 20% by weight of macrocyclic oligoester, which can be fed at a rate of between about 200 kg/hr and about 1,500 kg/hr. For example, after solvent removal, the macrocyclic oligoester, which is substantially free from solvent, may be collected at a rate of between about 80 kg/hr to about 250 kg/hr. Pellets and pastilles of formulated and shaped macrocyclic oligoesters also can be produced at a similar rate.

### Definitions

The following general definitions may be helpful in understanding the various terms and expressions used in this specification.

As used herein, a "macrocyclic" molecule means a cyclic molecule having at least one ring within its molecular structure that contains 8 or more atoms covalently connected to form the ring.

As used herein, an "oligomer" means a molecule that contains 2 or more identifiable structural repeat units of the same or different formula.

As used herein, an "oligoester" means a molecule that contains 2 or more identifiable ester functional repeat units of the same or different formula.

As used herein, a "macrocyclic oligoester" means a macrocyclic oligomer containing 2 or more identifiable ester functional repeat units of the same or different formula. A macrocyclic oligoester typically refers to multiple molecules of one specific formula having varying ring sizes. However, a macrocyclic oligoester may also include multiple molecules of different formulae having varying numbers of the same or different structural repeat units. A macrocyclic oligoester may be a co-oligoester or a higher order oligoester, i.e., an oligoester having two or more different structural repeat units having an ester functionality within one cyclic molecule.

As used herein, "an alkylene group" means -CₙH₂ₙ-, where n ≥ 2.

As used herein, "a cycloalkylene group" means a cyclic alkylene group, -CₙH_{2n-x-,} where x represents the number of H's replaced by cyclization(s).

As used herein, "a mono- or polyoxyalkylene group" means [-(CH₂)ₘ-O-]ₙ-(CH₂)ₘ- , wherein m is an integer greater than 1 and n is an integer greater than 0.

As used herein, "a divalent aromatic group" means an aromatic group with links to other parts of the macrocyclic molecule. For example, a divalent aromatic group may include a meta- or para- linked monocyclic aromatic group (e.g., benzene).

As used herein, "an alicyclic group" means a non-aromatic hydrocarbon group containing a cyclic structure therein.

As used herein, "partially-crystallized macrocyclic oligomer" means a macrocyclic oligomer at least a portion of which is in crystalline form. Partially-crystallized macrocyclic oligomer may have various degrees of crystallinity ranging from 1% crystalline to 99% crystalline. Crystallinity imparts handleablility to the macrocyclic oligomer, enabling it to be shaped, for example.

As used herein, "a continuous process" means a process that operates on the basis of a continuous flow of materials into and/or materials out of the process.

As used herein, "a polyester polymer composite" means a polyester polymer that is associated with another substrate such as a fibrous or particulate material. Illustrative examples of particulate material are chopped fibers, glass microspheres, and crushed stone. Certain fillers and additives thus can be used to prepare polyester polymer composites. A fibrous material means more substrate, e.g., fiberglass, ceramic fibers, carbon fibers or organic polymers such as aramid fibers.

As used herein, "a fabric material" means any substrate useful in receiving macrocyclic oligoesters during production and formulation processes and in preparing prepregs of macrocyclic oligomers. Typically, fabric materials include fiber tow, fiber web, fiber mat, and fiber felt. The fabric materials may be woven or non-woven, unidirectional, or random.

### Macrocyclic Oligoesters

Macrocyclic oligoesters that may be processed according to processes of this invention include, but are not limited to, macrocyclic poly(alkylene dicarboxylate) oligomers typically having a structural repeat unit of the formula: 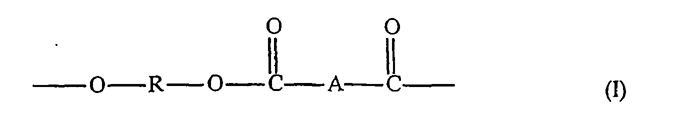 wherein R is an alkylene, a cycloalkylene, or a mono- or polyoxyalkylene group; and A is a divalent aromatic or alicyclic group.

Preferred macrocyclic oligoesters are macrocyclic oligoesters of 1,4-butylene terephthalate, 1,3-propylene terephthalate, 1,4-cyclohexylenedimethylene terephthalate, ethylene terephthalate, propylene terephthalate, and 1,2-ethylene 2,6-naphthalenedicarboxylate, and macrocyclic co-oligoesters comprising two or more of the above structural repeat units.

Synthesis of the macrocyclic oligoesters may be achieved by contacting at least one diol of the formula HO-R-OH with at least one diacid chloride of the formula: 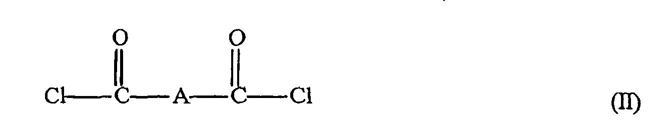 where R and A are as defined above. The reaction typically is conducted in the presence of at least one amine that has substantially no steric hindrance around the basic nitrogen atom. An illustrative example of such amines is 1,4-diazabicyclo[2.2.2]octane (DABCO). The reaction usually is conducted under substantially anhydrous conditions in a substantially water immiscible organic solvent such as methylene chloride. The temperature of the reaction typically is within the range of from about -25°C to about 25°C. See, e.g., U.S. Patent No. 5,039,783 to Brunelle et al*.*

Macrocyclic oligoesters also can be prepared via the condensation of a diacid chloride with at least one bis(hydroxyallcyl) ester such as bis(4-hydroxybutyl) terephthalate in the presence of a highly unhindered amine or a mixture thereof with at least one other tertiary amine such as triethylamine. The condensation reaction is conducted in a substantially inert organic solvent such as methylene chloride, chlorobenzene, or a mixture thereof. See, e.g., U.S. Patent No. 5,231,161 to Brunelle et al.

Another method for preparing macrocyclic oligoesters or macrocyclic co-oligoesters is the depolymerization of linear polyester polymers in the presence of an organotin or titanate compound. In this method, linear polyesters are converted to macrocyclic oligoesters by heating a mixture of linear polyesters, an organic solvent, and a transesterification catalyst such as a tin or titanium compound. The solvents used, such as *o*-xylene and *o*-dichlorobenzene, usually are substantially free from oxygen and water. See, e.g., U.S. Patent Nos. 5,407,984 to Brunelle et al. and 5,668,186 to Brunelle et al.

It is also within the scope of the invention to process macrocyclic co- and higher order oligoesters using the methods of the invention. Therefore, unless otherwise stated, an embodiment of a composition, article, or process that refers to macrocyclic oligoesters also includes embodiments utilizing macrocyclic co-oligoesters and higher order oligoesters.

### Isolation of Macrocyclic Oligoesters

In one aspect, the invention generally features processes for isolating a macrocyclic oligoester from a solution having a macrocyclic oligoester and a solvent in a manner that does not require use of an anti-solvent. In one embodiment, the process includes removing solvent to yield a macrocyclic oligoester substantially free from solvent. A solution including a macrocyclic oligoester and a solvent is provided. The solvent is then removed without the use of anti-solvent. In one embodiment, the solvent is removed under reduced temperature conditions. In another embodiment, the solvent is removed under elevated pressure conditions. In another embodiment, the solvent is removed under a combination of both elevated temperature and reduced pressure conditions. The macrocyclic oligoester substantially free from the solvent then typically is collected. In one embodiment, the solvent is continuously removed from the solution including a macrocyclic oligoester and a solvent. In another embodiment, the macrocyclic oligoester substantially free from the solvent is continuously collected.

There is no limitation to the concentration of macrocyclic oligoester in the solution. In one embodiment, the solution of a macrocyclic oligoester and a solvent (the input or feed solution) contains between about 1% and about 50% by weight macrocyclic oligoester. In other embodiments, the feed solution contains between about 3% and about 50%, between about 5% and about 40%, between about 5% and about 20%, between about 3% and about 10%, or between about 1% to about 3% by weight macrocyclic oligoester. The solution may contain one or two or more different solvents. "Solvent" used herein refers to the solvent or solvents contained in the feed solution.

Solvent removal may be carried out at an elevated temperature, at a reduced pressure, or both. In one embodiment, the feed solution is heated at an elevated temperature and a reduced pressure to remove the solvent from the solution. The resulting macrocyclic oligoester is substantially free from solvent. A macrocyclic oligoester is substantially free from solvent if the solvent content is less than 200 ppm. Preferably, the solvent content is less than 100 ppm. More preferably the solvent content is less than 50 ppm or less than 10 ppm.

The processing temperature and pressure for solvent removal are selected according to factors including the solvent to be removed, the solvent removal device(s) used, the desired time of purification, and the macrocyclic oligoester being isolated. In one embodiment, the step of removing solvent is conducted at a temperature within a range from ambient temperature to about 300°C. In other embodiments, the step of removing solvent is conducted from about 200°C to about 260°C, from about 230°C to about 240°C, or from about 180°C to about 200°C.

The pressure at which solvent removal is conducted can vary from atmospheric pressure to about 0.001 torr. In one embodiment, the pressure is within a range from 0.001 torr to about 0.01 torr. In other embodiments, the pressure is within a range from atmospheric pressure to about 10 torr, from about 10 torr to about 5.0 torr, from about 5.0 torr to about 1.0 torr, from about 1.0 torr to about 0.1 torr, or from about 0.1 torr to about 0.01 torr.

Solvent removal may be accomplished in almost any apparatus, e.g., vessels or devices or a combination of apparatus. Non-limiting examples of solvent removal apparatus that may be employed include a rising film evaporator, a falling film stripper, a thin film evaporator, a wiped film evaporator, a molecular still, a short path evaporator, a centrifuge, and a filter. The terms evaporator and stripper may be used interchangeably. In one embodiment, the rising film evaporator may be a tubular heat exchanger. A rising film evaporator is an apparatus used to vaporize part or all of the solvent from a solution where the solution is introduced to the bottom of the evaporator. A falling film stripper is an evaporative device for the removal of vapors from solution, where the solution is introduced to the top of the apparatus and travels to the bottom of the apparatus. A thin film evaporator is an apparatus that generates and exposes a thin film of material for evaporation and has the vapor condenser outside of the evaporator. A wiped film evaporator is an apparatus that generates and exposes a thin film of material to wiping or agitation to provide evaporation. A short path evaporator generates and exposes a thin film of material for evaporation and has the vapor condenser inside the evaporator. In some embodiments, the short path evaporator exposes the thin film to wiping or agitation to provide evaporation. A molecular still is an apparatus that utilizes a condenser inside the body of the still. One or more solvent removal device may be employed in accordance with the invention. In one embodiment, each solvent removal apparatus used in the process removes between about 80% and about 90% of the solvent. In one embodiment, multiple solvent removal apparatus are employed to achieve the desired dryness in the macrocyclic oligoester substantially free from solvent.

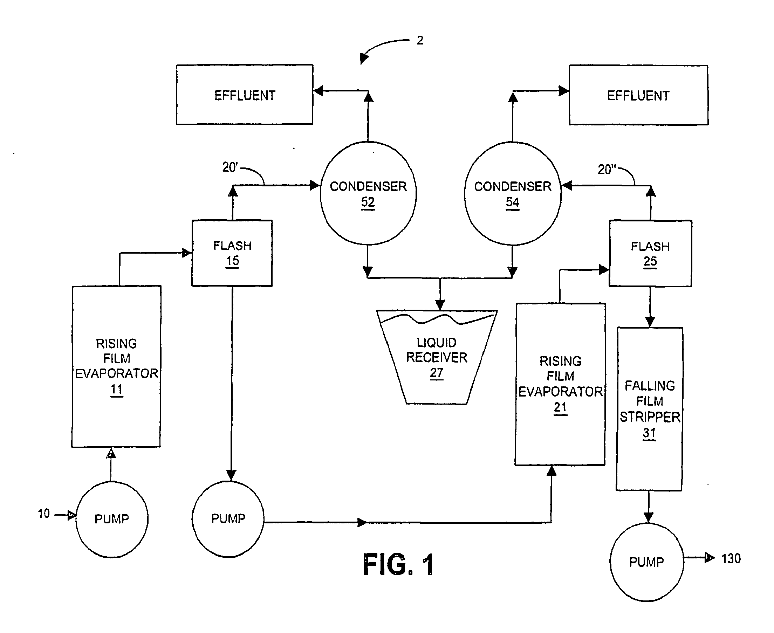

FIG. 1 schematically illustrates one embodiment of a solvent removal system **2.** An input solution **10** is pumped into a rising film evaporator **11.** As the input solution travels up the first rising film evaporator **11,** some of the solvent vaporizes and is separated from the solution. This solution and the vapor then travels through a flash device **15.** A flash device is an apparatus that is used to separate the liquid and the gas phase. The liquid phase solution then is pumped into a second rising film evaporator **21.** After traveling through another flash device **25,** the vapor phase solvent that is removed from flash devices **15** and **25** is pumped through paths **20'** and **20",** respectively, and is condensed in condensers **52** and **54.** The condensers **52** and **54** change any vapor phase solvent in paths **20'** and **20"** into a liquid phase. Optionally, effluent containing removed solvent may be discharged from condensers **52** and **54.** The condensed solvent is then collected in the liquid receiver **27.** The solution containing macrocyclic oligoester is pumped from flash device **25** into a falling film stripper **31.** In one embodiment, the vapors removed in the falling film stripper **31** also travel through the flash device **25.** An output product **130,** which is substantially free from solvent, is pumped out of the falling film stripper **31.** In one embodiment, the output product **130** is molten.

Substantially all of the solvent in the input solution can be removed from the macrocyclic oligoester to form a macrocyclic oligoester substantially free from solvent. In one embodiment, the macrocyclic oligoester substantially free from solvent may contain about 200 ppm or less of solvent. In other embodiments, the macrocyclic oligoester substantially free from solvent may contain about 100 ppm or less of solvent, about 50 ppm or less of solvent, and about 10 ppm or less of solvent. The amount of solvent remaining in the macrocyclic oligoester substantially free from solvent may be measured using chromatographic techniques such as gas chromatography, GCMS, or HPLC.

In determining an appropriate solvent stripping system to employ in a particular process, factors that need to be considered include the concentration of macrocyclic oligoester in the feed solution, the desired dryness and/or purity of the product, the solvent to be removed, and the desired length of time for solvent removal. For example, starting with a relatively dilute feed solution (i.e., low percentage of macrocyclic oligoester), more solvent removal steps and/or time may be necessary to produce a substantially solvent free macrocyclic oligoester. Conversely, a concentrated feed solution of macrocyclic oligoester may require fewer solvent removal steps and/or time.

Generally and in one embodiment, solvent is removed from an input solution by exposing the input solution to an elevated temperature and a reduced pressure in a first rising film evaporator. The input solution then travels to a second rising film evaporator where it is exposed to an elevated temperature and a reduced pressure. Finally, the input solution travels to a falling film stripper and a macrocyclic oligoester substantially free from solvent is collected from the falling film stripper.

In another general embodiment, solvent is removed from an input solution by exposing the feed solution to an elevated temperature and a reduced pressure in a first rising film evaporator. The input solution then travels through a first flash device. The solvent that is removed in the first rising film evaporator and the first flash device travels to a first condenser and the remainder of the input solution travels to a second rising film evaporator where it is exposed to an elevated temperature and a reduced pressure. The input solution then travels through a second flash device. The solvent that is removed in the second rising film evaporator and the second flash device travels to a second condenser. The solvent that has traveled through the first condenser and the second condenser is transported to a liquid receiver. The remainder of the input solution and the solvent travels to a falling film stripper. Optionally, the sparger may operate at the same time as the falling film stripper. Alternatively, a sparger removes gasses and vapors from the input solution after it has traveled through the stripper. Thereafter, a macrocyclic oligoester substantially free from solvent is collected.

When preparing macrocyclic oligoesters by depolymerizing linear polyesters, dilute conditions may be desired to promote cyclization and to increase the yield of macrocylcic oligoesters. As a result, the macrocyclic oligoester solution (e.g., the product solution of a depolymerization reaction) may be dilute (e.g., a 1% by weight macrocyclic oligoester solution).

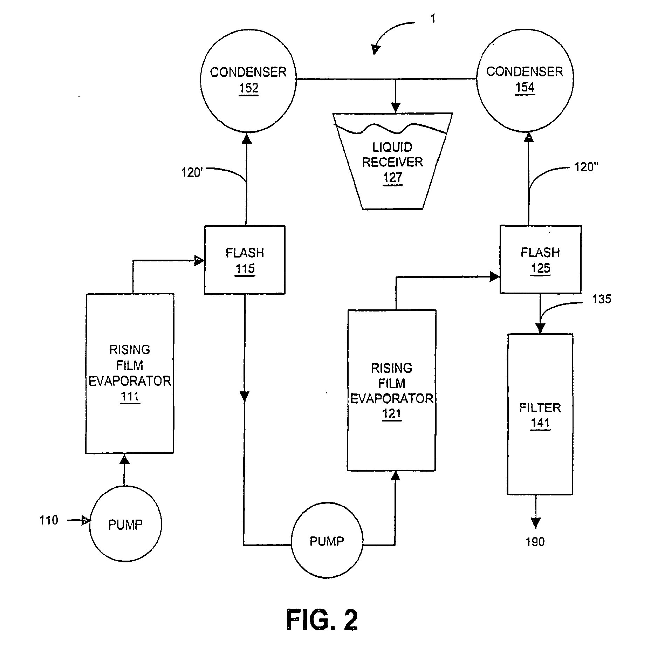

FIG. 2 schematically illustrates an embodiment of a system 1 for solvent removal that is typically employed where the solution is dilute (e.g., less than about 3% by weight macrocyclic oligoester). A linear polyester depolymerization reaction product solution (i.e., the input solution) **110** is pumped into a rising film evaporator **111.** Some of the solvent in the solution transitions into the vapor phase as it travels up the rising film evaporator **111** and it then travels though a flash device **115.** The solution then is pumped into a second rising film evaporator **121.** Thereafter the solution travels through another flash device **125.** The solution that exits the flash device **125** travels along path **135** and has a higher macrocyclic oligoester concentration (e.g., an increase from about 1% to about 3 %). The vapor phase solvent that is removed from flash devices **115** and **125** travels along paths **120'** and **120",** is condensed in condensers **152** and **154,** and is collected in a liquid receiver **127.** The macrocyclic oligoester solution that exits the flash device **125** then travels along path **135** to a filter **141,** which removes any remaining linear polyester and/or catalyst from the depolymerization reaction product solution. The filter **141** may be, for example, a Niagara filter or a Sparlder filter. A Niagara filter is a multiple tray filter available from Baker Hughes Corporation (Houston, TX). Similarly, a Sparlder filter is a multiple tray filter apparatus available from Sparkler Filters, Inc. (Conroe, TX). In one embodiment, a centrifuge is employed alternatively or in addition to the filter **141.** A resulting output solution **190** exiting filter **141** may become the input solution in the next solvent removal step.

The output solution **190** may have a macrocyclic oliogester concentration of about 3%. In one embodiment, the rising film evaporator **111** is held at a temperature between about 180°C-185°C at atmospheric pressure. In another embodiment, the rising film evaporator **121** is held at temperature between about 180°C-185°C at atmospheric pressure. In other embodiments, each rising film evaporator **111** and **121** is held at a temperature between about 120°C to 280°C at a pressure ranging from about 0.001 torr to about atmospheric pressure.

Referring again to FIG. 1, when the concentration of macrocyclic oligomer input solution **10** is about 3%, two additional rising film evaporators (not shown) may be placed in series between the first rising film evaporator **11** and the second rising film evaporator **21.** The two additional rising film evaporators may employ similar conditions as the first rising film evaporator **11** and use steam to heat the macrocyclic oligomer and the solvent (e.g., at about 150°C under a pressure of about 100 torr).

In one embodiment, the rising film evaporator employs steam to heat the solution to a temperature between about 120°C to 200°C. In yet another embodiment, the rising film evaporator employs hot oil to heat the solution to between about 200°C to about 280°C. The rising film evaporators may be operated at pressures ranging from about 0.001 torr to about atmospheric pressure. In one embodiment, between about 80% and about 90% of the solvent in the input solution is removed by each rising film evaporator. Where the input solution has a relatively low concentration of macrocyclic oligoester, multiple rising film evaporators may be employed in multiple steps. In one embodiment, multiple solvent removal apparatus are employed to achieve the desired dryness in the macrocyclic oligoester substantially free from solvent.

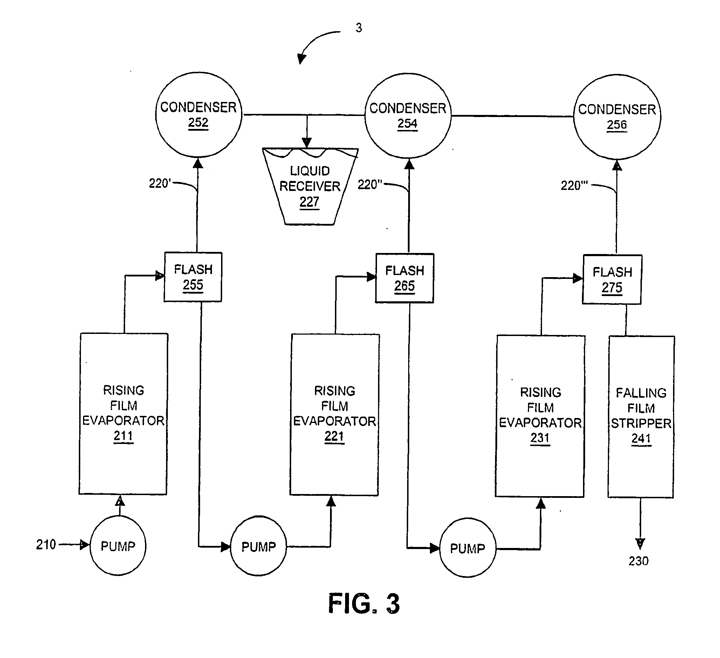

FIG. 3 schematically illustrates another embodiment of a solvent removal system **3.** The system shown in FIG. 3 may be used alone or in combination with that of FIG. 2. An input solution **210** is pumped into a first rising film evaporator **211.** Thereafter, the solution travels through a first flash device **255.** Condenser **252** captures the vaporized solvent that is removed in the first rising film evaporator **211** and the first flash device **255.** The solution then travels through a second rising film evaporator **221** to a second flash device **265.** Condenser **254** captures the vaporized solvent that is removed in the second rising film evaporator **221** and the second flash device **265.** The solution then travels through a third rising film evaporator **231.** Subsequently, the solution travels through a third flash device **275.** Condenser **256** captures the vaporized solvent that is removed in the third rising film evaporator **231** and the third flash device **275.** After traveling through the third flash device **275,** the solution travels through the falling film stripper **241.** A macrocyclic oligoester output product **230** substantially free from solvent is pumped out of the falling film stripper **241.** In one embodiment, the macrocyclic oligoester **230** is in a molten state. The vaporized solvent that is removed from flash device **255, 265,** and **275** travels along paths **220', 220",** and **220'''** and is condensed in condensers **252, 254** and **256,** and is collected in the liquid receiver **227.**

In another embodiment, the first rising film evaporator has about 20 square feet of evaporation surface area and is maintained at about atmospheric pressure and a temperature of about 185°C. The second rising film evaporator has about 5 square feet of evaporation surface area and is maintained at a pressure of about 1 torr and at a temperature ranging between about 185°C and about 190°C. The third rising film evaporator has about 1 square foot of evaporation surface area and is maintained at a pressure of about 1 torr and at a temperature ranging between about 185°C and about 190°C. In this embodiment, the first rising film evaporator, having a relatively large evaporation surface area and being run at atmospheric pressure, typically removes the bulk of solvent from the input solution.

Generally and in one embodiment of the invention, solvent is removed from an input solution of a macrocyclic oligoester by exposing the input solution to an elevated temperature and a reduced pressure in a first short path evaporator. A short path evaporator is used to vaporize part or all of the solvent from a solution. A short path evaporator can operate at a low pressure because the condenser is located inside of the evaporator. The input solution may then travel to a second short path evaporator where it is exposed to an elevated temperature and a reduced pressure.

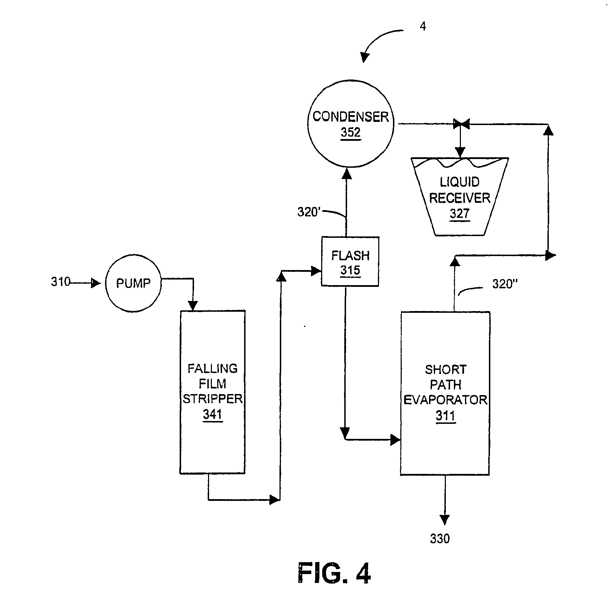

FIG. 4 schematically illustrates another embodiment of a solvent removal system **4**. An input solution **310** of a 3% by weight macrocyclic oligoester solution is pumped into the top of a falling film stripper **341.** Thereafter, the solution travels through a flash device **315.** The solvent that is vaporized in the falling film stripper **341** and the first flash device **315** travels through a path **320'** to a condenser **352**, and is removed from the solution. The solvent that has traveled through the condenser **352** is transported to a liquid receiver **327**. The solution travels to the short path evaporator **311.** In the short path evaporator **311** the solution is exposed to an elevated temperature and reduced pressure. The solvent that is vaporized in the short path evaporator **311** is condensed within the short path evaporator **311** and removed from the solution. The solvent removed within the short path evaporator **311** is transported through a path **320"** to a liquid receiver **327.** A macrocyclic oligoester output product **330** substantially free from solvent exits the short path evaporator **311**. In one embodiment, the macrocyclic oligoester **330** is in a molten state. A macrocyclic oligoester substantially free from solvent is collected from the short path evaporator **311**.

In one exemplary embodiment the input solution **310** of macrocyclic oligoester is heated to a temperature of about 180°C and is pumped into the top of the falling film stripper **341** at a rate of about 5900 kg/hr. The falling film stripper **341** is maintained at a temperature of about 180°C and at about atmospheric pressure. The solution exits the bottom of the falling film stripper **341** at a temperature of about 180°C. The solution enters the flash device **315**, which is held at atmospheric pressure and at a temperature of about 180°C. The solution exiting the flash device **315** that enters the short path evaporator **311** is at a temperature of about 180°C. The short path evaporator **311** has 2.4 m² of surface area, is held at a temperature of about 210°C and at a pressure of about 5 torr. The macrocyclic oligoester output product **330** exits the short path evaporator **311** at a rate of about 181 kg/hr and at a temperature of about 210°C. The output product **330** contains less than 100 ppm of solvent. A suitable falling film stripper **341,** flash device **315**, and short path evaporator **311** that may be employed in accordance with this exemplary embodiment are available from Incon Processing Technology (Batavia, IL).

In one embodiment, a compressor may be employed in place of a condenser. In another embodiment, the compressed gas or the condensed gas exiting the compressor or the condenser, respectively, may be employed as the heat input to one or more of the stripping apparatus and/or the evaporating apparatus. For example, where a shell and tube heat exchanger is employed, the compressed gas exiting a compressor may be fed to the shell side of the heat exchanger.

Generally, where short path evaporators have been employed in the solvent removal process, a sparger may not be necessary to obtain a macrocyclic oligoester substantially free from solvent. Short path evaporators can operate effectively under lower vacuum and at lower temperature conditions, thereby potentially saving energy costs. Also, the time required by the sparging step and the cost of maintaining sparging equipment are avoided when short path evaporators are employed.

Systems, apparatus, and equipment that may be employed or adapted to perform the processes described herein are commercially available, for example, from Artisan Industries Inc. of Waltham, MA and from LCI of Charlotte, NC. Suitable rising film evaporators include heat exchangers available from Troy Boiler (Albany, NY). Suitable falling film strippers, condensers and flash devices may be supplied by Artisan Industries Inc. (Waltham, MA) and Incon Processing Technology (Batavia, IL). Suitable short path evaporators are available from Incon Processing Technology (Batavia, IL). Suitable liquid receivers are available from suppliers including, Artisan Industries Inc. (Waltham, MA)and Incon Processing Technology (Batavia, IL.)

### Shaping Macrocyclic Oligoesters

In another aspect, the invention features a process for shaping a partially-crystallized macrocyclic oligoester. This process includes providing a substantially solvent-free molten macrocyclic oligoester. The substantially solvent-free molten macrocyclic oligoester is sheared to form a partially-crystallized macrocyclic oligoester, which can be shaped.

In one embodiment, the substantially solvent-free molten macrocyclic oligoester is continuously sheared to form a partially-crystallized macrocyclic oligoester. In another embodiment, shaping of the partially-crystallized macrocyclic oligoester is continuous. In yet another embodiment, the molten macrocyclic oligoester is continuously sheared and the partially-crystallized macrocyclic oligoester is continuously shaped.

Once substantially free from solvent, the macrocyclic oligoester, which may be a molten liquid at the solvent-removal temperature, is cooled and solidified into a usable form. When molten macrocyclic oligoester (such as macrocyclic (1,4-butylene terephthalate)) is cooled quickly, it is typically amorphous. In its amorphous state, the macrocyclic oligoester is sticky and "droplets" tend to agglomerate into a large mass. Amorphous macrocyclic oligoester also absorbs water from the atmosphere, which can be detrimental to subsequent processing.

Shear-induced partial-crystallization is used to facilitate crystallization of the macrocyclic oligoester. According to embodiments of the invention, an extruder, a scraped surface crystallizer, and/or a shear mixer are employed to partially-crystallize the product to form a partially-crystallized macrocyclic oligoester. A shear mixer includes any crystallizer that facilities crystallization by shear mixing. The extruder may be employed to extrude the macrocyclic oligoester at a temperature below the melting point of the macrocyclic oligoester, thereby forming a partially-crystallized macrocyclic oligoester. Shearing may include shearing, cooling, or shearing and cooling simultaneously.

Suitable product forms (e.g., pellets, pastilles, flakes, and prepregs) that are stable in the environment and easy to handle may be obtained by these methods. The partially-crystallized macrocyclic oligoester then may be collected. The collection may be continuously performed depending on the application.

Two or more processes of the invention may be carried out simultaneously. In one embodiment, an extruder removes solvent from the solution of macrocyclic oligoester to form a substantially solvent-free molten macrocyclic oligoester that the extruder shears to form a partially-crystallized macrocyclic oligoester that is shaped into a pellet.

The macrocyclic oligoester may be sheared at a temperature that is lower than the melting point of the macrocyclic oligoester. In one embodiment, the shearing step is conducted at a temperature between about 100°C and about 165°C. In another embodiment, the shearing step is conducted at a temperature between about 145°C and about 155°C.

Additionally, one or more of various additives and fillers can be incorporated into the macrocyclic oligoester, before, during or after solvent removal to yield a fully formulated product. For example, in the manufacture of an article, various types of fillers may be included. Filler often is included to achieve a desired property, and may be present in the resulting polyester polymer. The filler may be present to provide stability, such as chemical, thermal or light stability, to the blend material or the polyester polymer product, and/or to increase the strength of the polyester polymer product. A filler also may provide or reduce color, provide weight or bulk to achieve a particular density, provide flame resistance (i.e., be a flame retardant), be a substitute for a more expensive material, facilitate processing, and/or provide other desirable properties as recognized by a skilled artisan.

Illustrative examples of fillers are, among others, fumed silicate, titanium dioxide, calcium carbonate, chopped fibers, fly ash, glass microspheres, micro-balloons, crushed stone, nanoclay, linear polymers, and monomers. One or more fillers may be added before, during, or after the polymerization reaction between a macrocyclic oligoester and a cyclic ester. For example, fillers may be added to a substantially solvent-free macrocyclic oligoester. Optionally the filler may be added when the substantially solvent-free macrocyclic oligoester is in molten form. Also, fillers can be used to prepare polyester polymer composites.

In some embodiments, additional components (e.g., additives) are added to the macrocyclic oligoesters. Illustrative additives include colorants, pigments, magnetic materials, anti-oxidants, UV stabilizers, plasticizers, fire-retardants, lubricants, and mold releases. In other embodiments, one or more catalysts are added to the macrocyclic oligoester. Exemplary catalysts that may employed in accordance with the invention are described below.

### Formulating Macrocyclic Oligoesters

In another aspect, the invention features processes for formulating macrocyclic oligoesters and processes for making prepregs from macrocyclic oligoesters and polymerization catalysts.

In one embodiment, a mixture of a molten macrocyclic oligoester and a polymerization catalyst substantially free from solvent is provided. The mixture of the molten macrocyclic oligoester and polymerization catalyst is deposited onto a fabric material to form a prepreg. In one embodiment, the molten macrocyclic oligoester and polymerization catalyst are partially-crystallized prior to being deposited onto the fabric material.

A mixture of a molten macrocyclic oligoester and a polymerization catalyst substantially free from solvent may be continuously provided. The mixture of the macrocyclic oligoester and the polymerization catalyst may be partially crystallized. In one embodiment, the mixture is continuously partially crystallized. The partially-crystallized mixture of the macrocyclic oligoester and the polymerization catalyst then may be deposited onto a fabric material. In another embodiment, the partially-crystallized mixture is continuously deposited onto a fabric material.

In other embodiments, a macrocyclic oligoester (e.g., pellets) is fed to a hot mixing device (e.g., an extruder or a Readco mixer) with other solid or liquid additives (e.g., stabilizers or polymerization catalysts) with or without fillers. The mixing device partially melts the macrocyclic oligoester into a paste to enhance mixing and flow. The formulated product, which remains partially crystalline, then is formed into shapes such as pellets, flakes, pastilles, and/or applied directly to a fabric material to make a prepreg. This method typically avoids the problems of handling amorphous macrocyclic oligoester.

In yet other embodiments, the partially-crystallized mixture of molten macrocyclic oligoester and polymerization catalyst is deposited onto a fabric material. In certain embodiments the molten macrocyclic oligoester and polymerization catalyst are shear mixed in a shear mixer; alternatively, they may be processed in an extruder. The shear-mixing may be conducted at a temperature between about 145°C and about 155°C. The fabric material(s) may be selected from the group of fiber tow, fiber web, fiber mat, felt, non-woven material, and random and woven material.

Prior to partial-crystallization, the molten macrocyclic oligoester may contain less than about 200 ppm of solvent. Preferably, the molten macrocyclic oligoester contains less than about 100 ppm of solvent. More preferably, the molten macrocyclic oligoester contains less than about 50 ppm of solvent or less than about 10 ppm of solvent.

The partially-crystallized mixture of the macrocyclic oligoester and the polymerization catalyst may be deposited onto the fabric material in a pre-selected array. In addition, the fabric material having the mixture of macrocyclic oligoester and polymerization catalyst deposited thereon may be formed into a desired shape, for example, an autobody panel shape. One or more additives may be added to the molten macrocyclic oligoester. Exemplary additives may be selected from the group of colorants, pigments, magnetic materials, anti-oxidants, UV stabilizers, plasticizers, fire-retardants, lubricants, and mold releases.

In one embodiment, the molten macrocyclic oligoester and polymerization catalyst are partially-crystallized prior to being deposited onto the fabric material. The mixture of molton macrocyclic oligoester and polymerization catalyst may be partially-crystallized by, for example, shear mixing. In certain embodiments, shear mixing is conducted within a temperature range between about 145°C and about 155°C. In other embodiments the mixture of molton macrocyclic oligoester and polymerization catalyst is partially-crystallized by extrusion, which is often conducted within a temperature range between about 145°C and about 155°C.

The partially-crystallized mixture of macrocyclic oligoester and polymerization catalyst may be deposited onto the fabric material in discrete droplets of a selected size according to a pattern of a pre-selected array. In certain embodiments, the molten macrocyclic oligoester is mixed with one or more additive(s) and/or filler(s). The fabric material may be selected from the group of fiber tow, fiber web, fiber mat, felt, non-woven material, random, and woven material. The fabric material employed in a prepreg may vary depending on the end use application of the prepreg. Also, the fiber used to make the fiber material, or any fiber sizing agents or other agents present on the fiber material, may impact the suitability of the fiber material for use in a prepreg. For example, some catalysts and/or macrocyclic oligoester and polymerization catalyst mixtures may interact with the fibers and/or any sizing or other agents that are present on the fabric material.

In some embodiments, the partial crystallization step occurs continuously. In other embodiments, the partially-crystallized mixture of macrocyclic oligoester and the polymerization catalyst is continuously deposited on the fabric material. In still other embodiments, the process of making the prepreg is continuous whereby the mixture of a molten macrocyclic oligoester and a polymerization catalyst, which is substantially free from solvent, is continuously provided, continuously partially-crystallized, then continuously deposited onto a fabric material.

In another embodiments, the process of solvent removal and the process of prepreg formation are combined, creating a continuous process from the feed solution of a macrocyclic oligoester to formation of prepregs of macrocyclic oligoester substantially free from the solvent. The prepregs may contain one or more additives and a polymerization catalyst. Such continuous processes may provide advantages in many aspects such as in reducing energy cost and processing time and optimizing equipment usage.

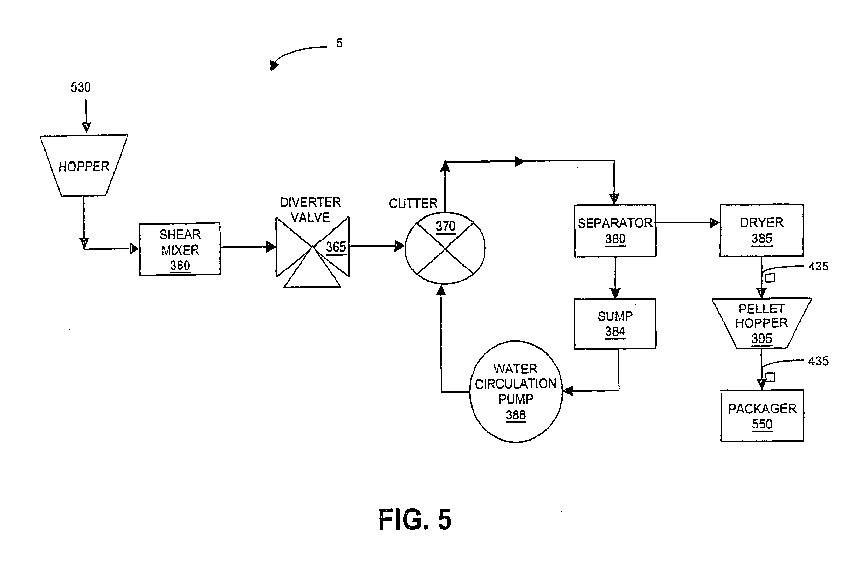

FIG. 5 schematically illustrates one embodiment of a process **5** for making pellets from a molten product with an underwater pelletizer. In this embodiment, a molten macrocyclic oligoester **530,** which is substantially free from solvent, is fed into a shear mixer **360.** The shear mixer **360** is connected to a temperature control loop (not shown). The shear mixer **360** may be a Readco mixer (York, PA), which is like a twin screw extruder, but less heavy duty. The molten macrocyclic oligoester within the shear mixer **360** is typically cooled to a temperature between about 80°C and about 140°C, preferably between about 130°C and about 140°C. By lowering the temperature in the shear mixer **360,** the macrocyclic oligomer is crystallized partially and is paste-like. Generally, the partially-crystallized macrocyclic oligomer employed to make pellets **435** measures between about 3000 cp. (centipoise) and about 5000 cp., which typically indicates that about 30% of the macrocyclic oligomer is crystallized.

The partially-crystallized macrocyclic oligomer travels from the shear mixer **360** to a diverter valve **365.** The diverter valve **365** may be used to divert the product from the process to, for example, a bucket when the pelletizer starts up. The diverter **365** typically is used to ensure that the partially-crystallized macrocyclic oligoester is traveling to the upstream cutter **370** at a minimum velocity. A suitable diverter **365** and a suitable cutter **370** may be available from Gala Industries (Eagle Rock, VA), Incon Processing Technology (Batavia, IL), and/or Artisan Industries Inc. (Waltham, MA). After a minimum velocity is achieved, the partially-crystallized macrocyclic oligomer travels to the cutter **370.** At the cutter **370,** the partially-crystallized paste-like macrocyclic oligomer is cut into the shape of pellets in a slurry of water. One or more pellets may be cut by the cutter **370** at once. The pellets are then removed from the slurry of water into a separator **380.** The separator **380** may be a screen, which may be a moving belt. A suitable separator **380** may be available from Gala Industries (Eagle Rock, VA), Incon Processing Technology (Batavia, IL), and/or Artisan Industries Inc. (Waltham, MA). Subsequently, the pellets **435** are dried in a dryer **385** and then transferred to a pellet hopper **395** and a packager **550.** The dryer **385** may be a fluid bed dryer available from Kason Corporation (Milburn, NJ). A suitable pellet hopper **395** and a suitable packager **550** may be available from Gala Industries (Eagle Rock, VA), Incon Processing Technology (Batavia, IL), and/or Artisan Industries Inc. (Waltham, MA). As depicted, the water that was separated from the pellets is recycled through a sump **384** and a water circulation pump **388.** A suitable sump **384** and a suitable circulation pump **388** may be available from Gala Industries (Eagle Rock, VA), Incon Processing Technology (Batavia, IL), and/or Artisan Industries Inc. (Waltham, MA).

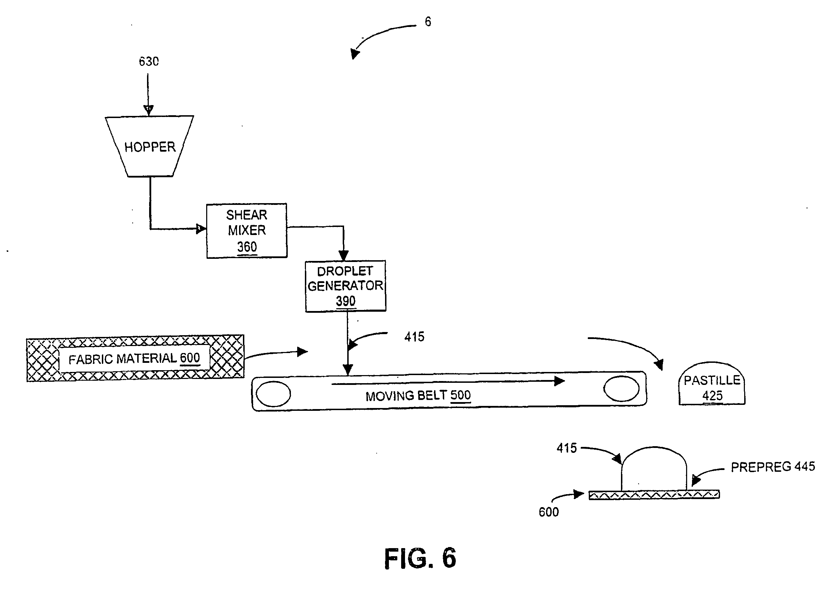

FIG. 6 schematically illustrates an embodiment of a process **6** for making either prepreg or pastille from a molten product utilizing a pastillation process. A molten macrocyclic oligoester **630,** which is substantially free from solvent, is fed into a shear mixer **360** that is connected to a temperature control loop (not shown) to control the temperature of the shear mixer **360.** The molten macrocyclic oligomer within the shear mixer **360** is typically cooled to a temperature between about 80°C and about 140°C, preferably between about 130°C and about 140°C.

In certain embodiments, the temperature control loop maintains the shear mixer **360** at a temperature of about 100°C. In other embodiments, the shear mixer **360** is an extruder. In yet other embodiments, the shear mixer **360** is a Readco mixer (York, PA), which is like a twin screw extruder, but less heavy duty.

By lowering the temperature in the shear mixer, the macrocyclic oligomer is partially crystallized and becomes paste-like. The temperature and/or the level of shear provided to produce the paste-like macrocyclic oligomer varies according to the composition of the macrocyclic oligomer, including the presence of any additives. Generally, the partially-crystallized macrocyclic oligomer employed to make pastilles measures between about 500 cp. and about 1000 cp., which typically indicates that it is between about 15% and about 20% crystallized. The molten macrocyclic oligomer may contain some residual solvent (e.g., between about 100 ppm and about 10 ppm) as the molten resin enters the shaping process at a temperature between about 150°C and about 200°C.

Both prepregs and pastilles can be made from the partially-crystallized and paste-like macrocyclic oligomer utilizing pastillation equipment. The partially-crystallized paste-like macrocyclic oligomer travels from the shear mixer **360** and enters a droplet generator **390.** The droplet generator **390** is employed to make desired sized droplets of macrocyclic oligoester. In one embodiment, a Sandvik Rotoformer available from Sandvik Process Systems of Totowa, NJ is employed to make droplets. When pastilles **425** are manufactured, the droplet generator **390** may drop pastilles **325** directly onto a moving belt **500.**

The moving belt **500** may be of any length and size and is typically between about 50 feet to about 100 feet in length. The bottom side of the moving belt **500** may be cooled, for example, by providing water underneath the moving belt **500.** The length of the moving belt 500 and the cooling method can be selected to cool the pastilles **425** before the end of the moving belt **500.** In some embodiments, a scraping bar (not shown) is employed at the end of the moving belt **500** to remove the pastilles **425** from the moving belt **500.** In one embodiment, a moving belt **500** available from Sandvik Process Systems of Totowa, NJ is employed.

When a prepreg **445** is manufactured, the droplet generator **390** may drop the material **415** (e.g., macrocyclic oligoester plus a polymerization catalyst) onto a fabric material **600** that is fed onto the moving belt **500.** The length of the moving belt **500** and any cooling method will be selected to cool the material **415** into the fabric material **600,** forming the prepreg **445.**

In some embodiments, an underwater pelletizer is used for making pellets. In For example, a Gala type underwater pelletizer (available from Gala Industries, Inc. of Eagle Rock, VA) may be used for making pellets. Alternatively, a pastillator may be used for forming pastilles. For example, a Sandvik Rotoformer (available from Sandvik Process Systems of Totowa, NJ) may be used to form pastilles.

In yet another aspect of the invention, the process of solvent removal and the process of shaping a partially-crystallized macrocyclic oligoester are combined, creating a continuous process from feeding a solution of a macrocyclic oligoester to shaping the macrocyclic oligoester. For example, in one embodiment, the process of solvent removal and the process ofpastillation may be combined, thereby creating a continuous process from input solution of a macrocyclic oligoester to pastilles of macrocyclic oligoester substantially free from the solvent. In one embodiment, a solution of macrocyclic oligoester is provided. During the solvent removal process, the temperature often is elevated to between about 180°C and about 200°C, and the pressure maintained between about atmospheric pressure and about 10 torr. In these embodiments, the solvent is continuously removed to produce a substantially solvent-free molten macrocyclic oligoester.

The substantially solvent-free molten macrocyclic oligoester may be sheared at a temperature below the melting point of the molten macrocyclic oligoester to form a partially-crystallized macrocyclic oligoester. The shearing temperature may be maintained at, for example, between about 145°C and about 155°C. Subsequently, the partially-crystallized macrocyclic oligoester may be formed into any desirable shapes including pellets, pastilles, and flakes.

Additives and fillers may be formulated with a macrocyclic oligoester or with a mixture of a macrocyclic oligoester and a catalyst. In one embodiment, the additive(s) and/or filler(s) are formulated with a macrocyclic oligoester while the latter macrocyclic oligoester is completely molten. In other embodiments, the additive(s) and/or filler(s) are formulated with a macrocyclic oligoester while the latter macrocyclic oligoester is partially molten and partially crystalline. In yet other embodiments, the additive(s) and/or filler(s) are formulated with a macrocyclic oligoester while the macrocyclic oligoester is completely crystalline. The formulated macrocyclic oligoester is prepared into a prepreg in the form of pastilles on a fabric material.

Pastille prepregs may be prepared from a blend material that includes macrocyclic oligoesters. In one embodiment, the invention relates to methods for preparing pastille thermoplastic prepregs based on a blend material that includes at least one macrocyclic oligoester and at least one polymerization catalyst.

Thermoplastic prepregs typically have been produced with the resin close to the fiber. If the melt viscosity of the resin is high, the resin needs to be close to the fiber in order to wet-out the fiber properly. This typically is the case with thermoplastic prepregs made using a hot melt method with thermoplastic powder, co-mingled tows of reinforcing fiber and thermoplastic fiber, or co-woven fabrics. These materials require a process which often includes three steps: 1) heating and melting the resin, 2) wetting out of the fiber and consolidating, and 3) cooling down and solidifying.

Macrocyclic oligoesters, as discussed above, melt to a low viscosity that may be many orders of magnitude lower than the viscosity of conventional thermoplastics. Thus, combining and wetting-out of macrocyclic oligoesters (when melted) with fillers and/or reinforcing fibers during the heating cycle of a process can be done much more easily than conventional thermoplastics. Hence, in prepreg fabrics made with macrocyclic oligoesters, the resin does not need to be distributed as close to the fiber (i.e., each and every fiber) as is needed for conventional thermoplastics. That is, resin can be placed at discrete locations, but melt and flow to wet-out the entire fabric when the resin is melted.

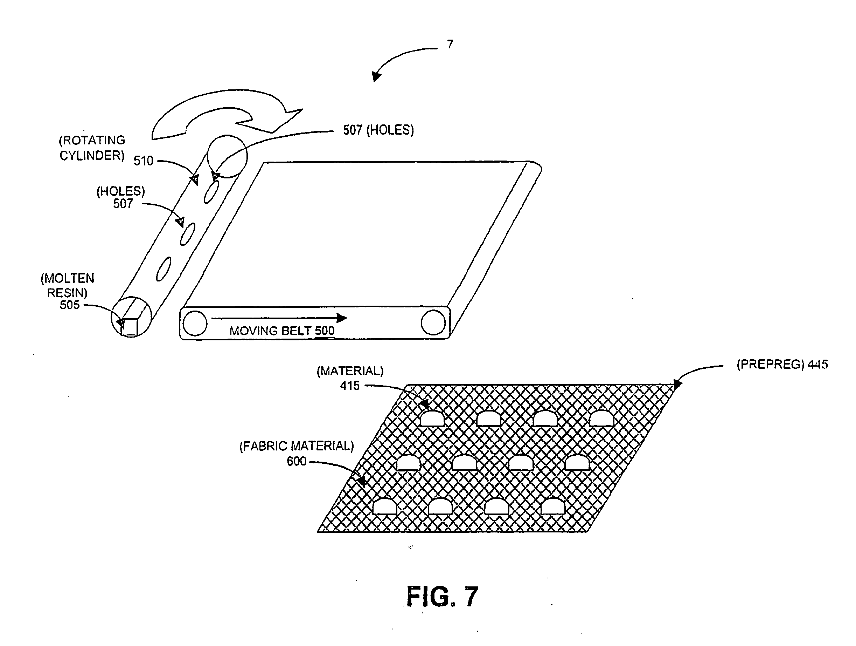

When a prepreg is made with a blend material that includes a macrocyclic oligoester, the blend material can be a one-part ready-to-use system with a catalyst already included. FIG. 7 illustrates one embodiment of the invention, a process 7 for making a prepreg **445** from a macrocyclic oligoester or a blend material of macrocyclic oligoester with one or more other components such as a polymerization catalyst. The process allows the making of a prepreg **445** that has the desired resin and fabric material in a pre-selected ratio. Such prepregs can simplify upstream processes employing prepregs.

Referring to FIG. 7, a blend material (e.g., a one-part system) is melted and applied to a reinforcing fabric **600** in discreet resin drops **415** and then cooled before significant polymerization takes place. The molten resin **505** is pumped into a channel in the bottom of a rotating cylinder **510** and comes out through the holes **507** in the cylinder each time the holes **507** line up with the channel. In one embodiment, a rotating cylinder **510** available from Sandvik Process Systems of Totowa, NJ is employed in the process. Consequently, liquid drops of resin fall at pre-determined intervals onto a moving belt **500** (e.g., a steel belt). These discrete resin drops **415** can be arranged in a pre-selected array (e.g., a pattern) so that the amount of resin is uniform per unit fabric area (if uniformity is desired) and is of a desired value. In one embodiment, the amount of resin per unit fabric area ranges from about 3% by weight resin to about 97% by weight resin. In another embodiment, the amount of resin per unit fabric area ranges from about 30% by weight resin to about 80% by weight resin.

The amount, pattern, and spacing of the dropped resin determine the "average" ratio of fabric material to resin before the resin is melted and distributed throughout the fabric material. There is no limitation as to the amount and pattern of the resin drops as long as the desired preregs are formed. The ratio of fabric material to resin may be uniform or varied across the prepreg and can be manipulated by controlling the size of each drop of resin and the space between them.

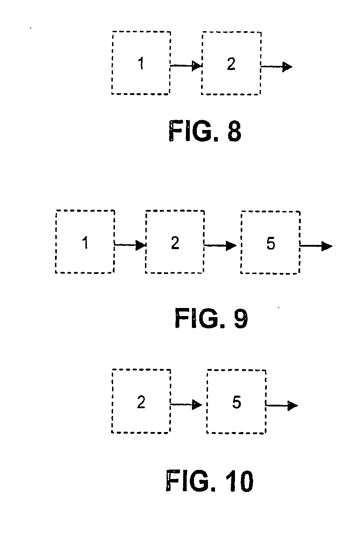

FIG. 8 illustrates a schematic flow diagram of an embodiment of a solvent removal system where the solvent removal system **1** illustrated in FIG. 2 is linked with the solvent removal system **2** illustrated in FIG. 1. According to this embodiment, which is typically employed where the linear polyester depolymerization reaction product solution (i.e., the input solution) is a dilute (e.g., about 1% by weight macrocyclic oligoester), input solution **110** is first processed though system **1** to yield a resulting output solution **190.** The solution **190** that is the product of system **1** typically contains about 3% by weight macrocyclic oligoester. The solution **190** enters system **2** as input solution **10.** The input solution **10** is processed thorough system **2** to yield an output product **130** substantially free from solvent.

FIG. 9 is a schematic flow diagram of an embodiment of a system for shaping macrocyclic oligoesters from a solution of macrocyclic oligoester and solvent. According to this embodiment, the linked solvent removal systems 1 and 2, described above, are further linked to the process 5 for making pellets from a molten product illustrated in FIG. 5. The input solution **110** is a dilute solution (e.g., about 1% by weight macrocyclic oligoester) which is first processed though system **1** to yield a resulting output solution **190.** The solution **190** that is the product of system **1** typically contains about 3% by weight macrocyclic oligoester. Solution **190** enters system **2** as input solution **10** and is processed thorough system 2 to yield an output product **130** substantially free from solvent. The output product **130** may be molten. Output product **130** enters process 5 as molten macrocyclic oligoester **530,** which is processed through system 5 to yield pellet **435.**

FIG. 10 is a schematic flow diagram of an embodiment of a system for shaping macrocyclic oligoesters from a solution of macrocyclic oligoester and solvent. According to this embodiment, the solvent removal system **2** is linked to the process **5,** described above, which make pellets from a molten product. According to this embodiment, the input solution **10** containing about 3% by weight macrocyclic oligoester is processed thorough system 2 to yield an output product **130** substantially free from solvent. In one embodiment, the output product **130** is molten. Output product **130** enters system **5** as molten macrocyclic oligoester **530,** which is processed through system **5** to yield pellet **435.**

Just as the processes in FIG. 8-10 can be linked to provide increased benefits, in other variations of such embodiments (not shown), alternative solvent removal system(s), for example, systems **1,2, 3,** and **4,** described above with reference to FIGS. 1-4, may be linked to one another and/or to processes for shaping macrocyclic oligoesters from a solution of macrocyclic oligoester and solvent, such as, for example, processes **5, 6,** and **7,** described above with reference to FIGS. 5-7. For example, referring again to FIG. 8, FIG. 9, and FIG. 10 the solvent removal system **2** can be replaced by system **3** (FIG. 3) or system **4** (FIG. 4). Referring still to FIG. 9, and FIG. 10, the shaping process **5** can be replaced by process **6** (FIG. 6).

The advantages of the above systems, processes and product prepregs include the ability to "drape" easily into a mold, the ability to flex without cracking and crumbling the resin drops, and the ability to use conventional pastillation equipment. Also, instead of placing the pellets on a conveyor belt, they are placed on a reinforcing fabric. In addition, the processes can be conducted isothermally (i.e. at constant temperature) and in a vacuum bag or in a compression molding press.

Catalysts may be formulated with macrocyclic oligoesters to prepare prepregs. Catalysts may be part of a blend material of macrocyclic oligoesters, see U.S. Patent No. 6,369,157, the entire contents of which is incorporated by reference herein, or may be added before or during the formulation processes described herein. Catalysts that may be employed in the invention include those that are capable of catalyzing a transesterification polymerization of a macrocyclic oligoester. As with state-of-the-art processes for polymerizing macrocyclic oligoesters, organotin, and organotitanate compounds are the preferred catalysts, although other catalysts may be used. Detailed description of polymerization catalysts can be found in commonly assigned U.S. Serial No. 09/754,943 entitled "Macrocyclic Polyester Oligomers and Processes for Polymerizing the Same" by Winckler *et al*., U.S. Serial No. 10/102,162 entitled "Catalytic Systems" by Wang, and U.S. Serial No. 10/040,530 entitled "Polymer-Containing Organo-Metal Catalysts" by Wang, the entire contents of which are incorporated by reference herein.

Illustrative examples of classes of tin compounds that may be used in the invention includes monoalkyltin(IV) hydroxide oxides, monoalkyltin(IV) chloride dihydroxides, dialkyltin(IV) oxides, bistrialkyltin(IV) oxides, monoalkyltin(IV) trisalkoxides, dialkyltin(IV) dialkoxides, trialkyltin(IV) alkoxides, tin compounds having the formula (II): 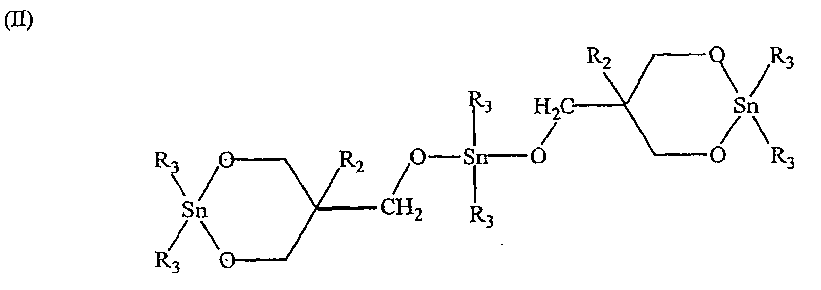 and tin compounds having the formula (III): 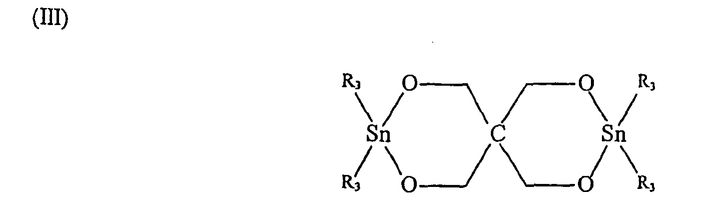 wherein R₂ is a C₁₋₄ primary alkyl group, and R₃ is C₁₋₁₀ alkyl group.

Specific examples of organotin compounds that may be used in this invention include dibutyltin dioxide, 1,1,6,6-tetra-n-butyl-1,6-distanna-2,5,7,10-tetraoxacyclodecane, n-butyltin(IV) chloride dihydroxide, di-n-butyltin(IV) oxide, dibutyltin dioxide, di-n-octyltin oxide, n-butyltin tri-n-butoxide, di-n-butyltin(IV) di-n-butoxide, 2,2-di-n-butyl-2-stanna-1,3-dioxacycloheptane, and tributyltin ethoxide. See, e.g., U.S. Patent No. 5,348,985 to Pearce et al. In addition, tin catalysts described in commonly owned U.S.S.N. 09/754,943 (incorporated herein by reference in its entirety) may be used in the polymerization reaction.

Titanate compounds that may be used in the invention include titanate compounds described in commonly owned U.S.S.N. 09/754,943. Illustrative examples include tetraalkyl titanates (e.g., tetra(2-ethylhexyl) titanate, tetraisopropyl titanate, and tetrabutyl titanate), isopropyl titanate, titanate tetraalkoxide. Other illustrative examples include (a) titanate compounds having the formula (IV): 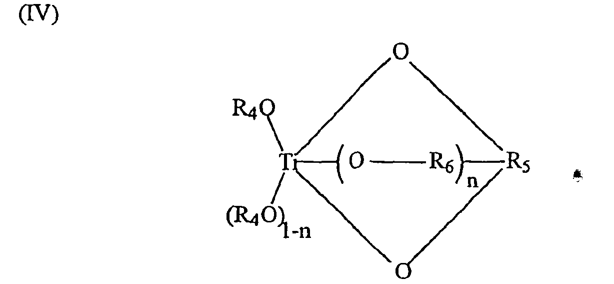 wherein each R₄ is independently an allcyl group, or the two R₄ groups taken together form a divalent aliphatic hydrocarbon group; R₅ is a C₂₋₁₀ divalent or trivalent aliphatic hydrocarbon group; R₆ is a methylene or ethylene group; and n is 0 or 1,
(b) titanate ester compounds having at least one moiety of the formula (V): 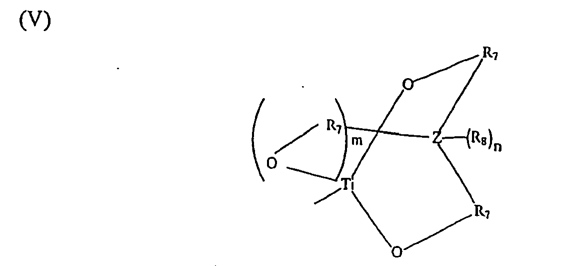 wherein each R₇ is independently a C₂₋₃ alkylene group; Z is O or N; R₈ is a C₁₋₆ alkyl group or unsubstituted or substituted phenyl group; provided when Z is O, m=n=0, and when Z is N, m=0 or 1 and m+n = 1, and
(c) titanate ester compounds having at least one moiety of the formula (VI): 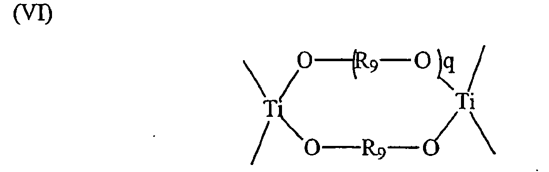 wherein each R₉ is independently a C₂₋₆ allcylene group; and q is 0 or 1.

The compositions and methods of the invention may be used to manufacture articles of various size and shape from various macrocyclic oligoesters. Exemplary articles that may be manufactured by the invention include without limitation automotive body panels and chassis components, bumper beams, aircraft wing skins, windmill blades, fluid storage tanks, tractor fenders, tennis rackets, golf shafts, windsurfing masts, toys, rods, tubes, bars stock, bicycle forks, and machine housings.

### Examples

The following examples are provided to further illustrate and to facilitate the understanding of the invention. These specific examples are intended to be illustrative of the invention.

### Example A

The macrocyclic oligoesters used in the following examples are the macrocyclic oligoesters of 1,4-butylene terephthalate. The macrocyclic oligoesters were prepared by heating a mixture of polyester linears, organic solvents, such as *o*-xylene and *o*-dichlorobenzene, which are substantially free from oxygen and water, and tin or titanium compounds as transesterification catalysts. See U.S. Patent No. 5,668,186 (incorporated herein by reference in its entirety).

### Example 1 Preparation of Macrocyclic (1,4-butylene terephthalate) Oligomer Pellets

Macrocyclic (1,4-butylene terephthalate) oligoester powder was fed at a rate of about 9 kg/hr through an extruder at about 120°C to melt into a paste and processed at a rate of about 9 kg/hr through a Gala underwater pelletizer available from Gala Industries (Eagle Rock, VA). No die freeze off was observed. The material cut cleanly on the die face. The pellets were strained out of the water and air dried to contain 80 ppm or less of water.

### Example 2 Macrocyclic (1,4-butylene terephthalate) Oligomer Pastilles

Macrocyclic (1,4-butylene terephthalate) oligomer powder containing less than 1,000 ppm solvent was melted in a tank at about 170°C and fed at a rate of 60 kg/hr to a Sandvik Rotoformer to form pastilles. No partial-crystallization was used. The pastilles were amorphous and agglomerated together. The macrocyclic (1,4-butylene terephthalate) oligomer was pastilled smoothly into pastilles.

### Example 3 Formulated Macrocyclic (1,4-butylene terephthalate) Oligomer Pastilles

Macrocyclic (1,4-butylene terephthalate) oligoester powder was melted and melt blended at a temperature between about 120°C and about 140°C with additives including a polymerization catalyst (0.33% by weight FASTCAT 4101 (Atofina, Philadelphia, PA)) and thermal stabilizers (0.4 % by weight IRGANOX 1010 (Ciba Specialty Chemicals Corporation, Tarrytown, NY)). The formulated product was then fed at a rate of about 45 kg/hr to the Sandvik Rotoformer to form pastilles, as in Example 2.

### Example 4 Formulated Macrocyclic (1,4-butylene terephthalate) Oligomer Pastilles on Glass Mat

Macrocyclic (1,4-butylene terephthalate) oligoester powder was melt blended with catalyst (0.33% by weight FASTCAT 4101 catalyst) and stabilizers (0.4 % by weight IRGANOX 1010) and pastilled onto glass mat attached to the Sandvik Rotoformer belt. The Macrocyclic (1,4-butylene terephthalate) oligoester contained less than 1000 ppm solvent. The weight of macrocyclic (1,4-butylene terephthalate) oligoester deposited onto an area of glass mat was controlled to between about 400 g/m² to about 800 g/m². The pastilles had a hemispherical shape and were about 7 mm in diameter, the pastilles were spaced about 15 mm apart from one another. The glass mat prepreg was flexible, with good adhesion of the macrocyclic (1,4-butylene terephthalate) oligoester pastilles. This prepreg mat can be cured to crystallize the macrocyclic (1,4-butylene terephthalate) oligoester to reduce moisture adsorption and tack. The prepreg was polymerized at a temperature of about 190°C to high molecular weight polyester (about 80,000 Dalton).

### Example 5 Solvent Removal via Stripping

A solution of macrocyclic (1,4-butylene terephthalate) oligoester in o-dichlorobenzene was fed to an Artisan evaporative stripper from Artisan Industries, Inc. (Waltham, MA.) A two-stage flash evaporator was operated at a temperature ranging between about 180°C and about 220°C and at a pressure ranging between about 10 torr and about atmospheric pressure to concentrate a 10% solution of macrocyclic (1,4-butylene terephthalate) oligomer to less than 100 ppm o-dichlorobenzene.

### Example 6 Solvent Removal via Evaporation and Stripping

An input solution of 3% by weight macrocyclic (1,4-butylene terephthalate) oligoester in o-dichlorobenzene solution was fed at a rate of about 6,045 kg/hr into a series of rising film evaporators and a falling film stripper available from Artisan Industries, Inc. to produce an output solution with solvent levels of less than 100 ppm at a rate of about 181 kg/hr.

In one embodiment, the input solution, having a temperature of about 65°C, is fed at a rate of about 6,045 kg/hr into a first rising film evaporator having an evaporation surface area of about 317 ft². The first rising film evaporator is held at temperature of about 180°C at atmospheric pressure. Thereafter, the solution exits the first rising film evaporator and enters a first flash device. The first flash device is held at temperature of about 180°C at atmospheric pressure. A first condenser captures the vaporized solvent that is removed in the first rising film evaporator and the first flash device.

The solution exits the first flash device and travels at a temperature of about 180°C to a second rising film evaporator. The second rising film evaporator has an evaporation surface area of about 81 ft² and is held at a temperature of about 193°C at atmospheric pressure. The solution exiting the second rising film evaporator has a temperature of about 193°C and enters a second flash device. The second flash device is held at a temperature of about 180°C at atmospheric pressure. A second condenser captures the vaporized solvent that is removed in the second rising film evaporator and the second flash device.

The solution exits the second flash device and travels to a third rising film evaporator. The third rising film evaporator has an evaporation surface area of about 21 ft² and is held at temperature of about 199°C at atmospheric pressure. Thereafter, the solution exits the third rising film evaporator at a temperature of about 199°C and enters a third flash device. The third flash device is held at a temperature of about 180°C at atmospheric pressure. A third condenser captures the vaporized solvent that is removed in the third rising film evaporator and the third flash device.

The solution exits the third flash device and travels to a fourth rising film evaporator. The fourth rising film evaporator has an evaporation surface area of about 8 ft² and is held at temperature of about 204°C at atmospheric pressure. Thereafter, the solution exits the fourth rising film evaporator at a temperature of about 204°C and enters a fourth flash device. The fourth flash device is held at a temperature of about 180°C at atmospheric pressure. A fourth condenser captures the vaporized solvent that is removed in the fourth rising film evaporator and the fourth flash device. Each of the four condensers employ cooling water to condense the vaporized solvent and bring the condensed solvent to a temperature of about 176°C.

The solution exits the fourth flash device and travels to a fifth rising film evaporator. The fifth rising film evaporator has an evaporation surface area of about 10 ft² and is held at temperature of about 226°C at a pressure of about 1 torr. Thereafter, the solution exits the fifth rising film evaporator at a temperature of about 226°C and enters the top of a falling film stripper. The falling film stripper is held at a temperature of about 226°C at a pressure of about 1 torr. A vacuum pump captures the vaporized solvent that is removed in the falling film stripper and the fifth rising film evaporator. The vacuum pump is held at about 0.5 torr. The vaporized solvent travels from the vacuum pump to a fifth condenser. The fifth condenser is sized at 75 ft² and employs cooling water to condense the vaporized solvent and bring the condensed solvent to a temperature of about 176°C.

Nitrogen from a nitrogen sparger is introduced to the solution traveling through the falling film stripper at a rate of about 9 kg/hr. After sparging, the macrocyclic oligoester product has a temperature of about 226°C and contains less than 100 ppm solvent. The macrocyclic oligoester exits the process at a rate of about 181 kg/hr.

Alternatively, a single flash device or a single condenser is employed in place of two or more of the flash devices and two or more of the condensers that are described. A single flash device may be employed in the place of the second, third, and fourth flash devices described above. A single flash device may house three distinct conduits for the solutions exiting the second, third, and fourth rising film evaporators. Such a flash device may have three conduits that are adjacent to one another. The flash device may also be constructed so that the conduit for the solution exiting the third rising film evaporator is placed inside the conduit for the solution exiting the second rising film evaporator, and the conduit for the solution exiting the fourth rising film evaporator is placed inside the conduit for the solution exiting the third rising film evaporator. A single condenser (e.g., a condenser with a 500 ft² area) may be employed in the place of the second, third, and fourth condenser described above.

Each of the patent and patent application documents disclosed hereinabove are incorporated by reference herein in their entirety.

Variations, modifications, and other implementations of what is described herein will occur to those of ordinary skill in the art without departing from the spirit and the scope of the invention as claimed.

## Claims

1. A process for shaping a macrocyclic oligoester, the process comprising the steps of:
(a) partially crystallizing macrocyclic oligoester comprising a structural repeat unit of formula (I): 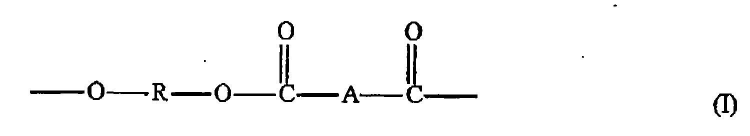 wherein R is an alkylene, a cycloalkylene, or a mono- or polyoxyalkylene group and A is a divalent aromatic or alicyclic group; and
(b) shaping the partially crystallized macrocyclic oligoester to form pellets, pastilles, flakes or prepreg.

2. A process as claimed in claim 1, wherein the process is continuous.

3. A process as claimed in claim 1 or 2, wherein the macrocyclic oligoester comprises a structural repeat unit selected from the group consisting of ethylene terephthalate, propylene terephthalate, 1,3-propylene terephthalate, 1,4-butylene terephthalate, 1,4-cyclohexylenedimethylene terephthalate, and 1,2-ethylene 2,6-naphthalenedicarboxylate.

4. A process as claimed in claim 1 or 2, wherein the macrocyclic oligoester comprises structural repeating unit 1,4-butylene terephthalate.

5. A process as claimed in any one of the preceding claims, further comprising the step of adding at least one additive to the macrocyclic oligoester, wherein the at least one additive is selected from the group consisting of a colorant, a pigment, a magnetic material, an antioxidant, a UV stabilizer, a plasticizer, a fire retardant, a lubricant, a mold release, fumed silicate, titanium dioxide, calcium carbonate, chopped fiber, fly ash, glass microspheres, micro-balloons, crushed stone, nanoclay, linear polymers, monomers, and catalyst.

6. A process as claimed in claim 5, wherein step (b) comprises shaping a mixture comprising the macrocyclic oligoester and a catalyst.

7. A process as claimed in any one of the preceding claims, wherein the step of partially crystallizing the macrocyclic oligoester comprises shearing, cooling, or shearing and cooling simultaneously.

8. A process as claimed in any one of the preceding claims, wherein the step of partially crystallizing the macrocyclic oligoester comprises mixing or extruding the macrocyclic oligoester.

9. A process as claimed in any of the preceding claims, wherein the step of partially crystallizing the macrocyclic oligoester is conducted at a temperature within a range of about 145°C and about 155°C.

10. A process as claimed in any one of the preceding claims, further comprising the steps of:
(i) prior to step (a), providing a solution comprising the macrocyclic oligoester and a solvent; and
(ii) removing the solvent.

11. A process as claimed in claim 10, wherein the macrocyclic oligoester has a solvent content less than 200 ppm following step (ii).

12. A process as claimed in claim 10, wherein the macrocyclic oligoester has a solvent content less than 50 ppm following step (ii).

13. A process as claimed in any one of claims 10 to 12, wherein step (ii) comprises removing the solvent at an elevated temperature, or at a reduced pressure, or both.

14. A process as claimed in claim 13, wherein step (ii) is conducted within a temperature range of about ambient temperature to about 300°C.

15. A process as claimed in claim 13, wherein step (ii) is conducted within a temperature range of about 180°C to about 200°C.

16. A process as claimed in any one of claims 13 to 15, wherein step (ii) is conducted within a pressure range of about 0.001 torr [0.13 Pa] to about 10 torr [1300 Pa].

17. A process as claimed in any one of claims 13 to 15, wherein step (ii) is conducted within a pressure range of about 1 torr [130 Pa] to about 10 torr [1300 Pa].

18. A process as claimed in any one of claims 13 to 17, wherein step (ii) comprises removing the solvent without using an anti-solvent.

19. A process as claimed in any one of claims 13,16,17 or 18, wherein step (ii) comprises removing the solvent at a temperature above 180°C.

20. A process as claimed in any one of claims 13,16,17 or 18, wherein step (ii) comprises removing the solvent at a temperature within a range from 120°C to 280°C.

21. A process as claimed in any one of claims 13,16, 17 or 18, wherein step (ii) comprises removing the solvent at a temperature within a range from 120°C to 200°C.

22. A process as claimed in any one of claims 13, 16, 17 or 18, wherein step (ii) comprises removing the solvent at a temperature within a range from 200°C to 280°C.

23. A process as claimed in any one of claims 13 to 22, wherein step (ii) comprises removing the solvent at a temperature above a melting point of the macrocyclic oligoester.

24. A process as claimed in any one of claims 10 to 23, wherein step (ii) and step (b) are combined.

25. A process as claimed in any one of the preceding claims, wherein step (b) comprises pelletizing the partially-crystallized macrocyclic oligoester.

26. A process as claimed in any one of the preceding claims, wherein step (b) comprises cutting the partially-crystallized macrocyclic oligoester in water.

27. A process as claimed in any one of the preceding claims, further comprising the step of collecting the product of step (b).

28. A process as claimed in any one of the preceding claims, wherein step (b) comprises forming the partially-crystallized macrocyclic oligoester into pellets in water.

29. A process as claimed in claim 28, wherein step (b) is conducted with an underwater pelletizer.

30. A process as claimed in claim 28 or 29, comprising the step of partially-crystallizing the macrocyclic oligoester prior to step (a) by cooling molten macrocyclic oligoester.

31. A process as claimed in claim 30, wherein the molten macrocyclic oligoester is cooled to a temperature between about 80°C and about 140°C.

32. A process as claimed in claim 30 or 31, wherein the partially-crystallized macrocyclic oligoester in step (b) has a viscosity between about 3000 cp and about 5000 cp.

33. A process as claimed in any one of claims 28 to 32, further comprising the step of removing the water with a dryer.

34. A process as claimed in any one of claims 1 to 24, wherein step (b) comprises pastillating the partially-crystallized macrocyclic oligoester using a droplet generator.

35. A process as claimed in claim 34, wherein the partially-crystallized macrocyclic oligoester enters the droplet generator at a temperature between about 150°C and about 200°C.

36. A process as claimed in claim 34 or 35, wherein the partially-crystallized macrocyclic oligoester in step (b) has a viscosity between about 500 cp and about 1000 cp.

37. A process as claimed in any one of claims 34 to 36, wherein step (b) comprises dropping a mixture comprising the macrocyclic oligoester and a catalyst onto a fabric to form a prepreg.

38. A shaped macrocyclic oligoester, formed by a process as claimed in any one of the preceding claims.

39. The shaped macrocyclic oligoester as claimed in claim 38, in the form of a pellet, a pastille, a flake, or a prepreg.

40. The shaped macrocyclic oligoester as claimed in claim 38, in the form of a pellet or a pastille.

## Patentansprüche

1. Verfahren zum Formen eines makrocyclischen Oligoesters, wobei das Verfahren die folgenden Schritte umfasst:
(a) Teilkristallisieren des makrocyclischen Oligoesters, der eine strukturelle Wiederholungseinheit der Formel (I) umfasst: 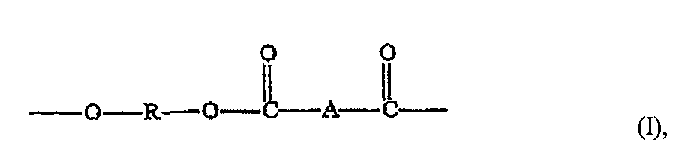 worin R eine Alkylen-, eine Cycloalkylen- oder eine Mono- oder Polyoxyalkylengruppe ist und A eine divalente aromatische oder alicyclische Gruppe ist; und
(b) Formen des teilkristallisierten makrocyclischen Oligoesters zur Bildung von Pellets, Pastillen, Flocken oder Prepreg.

2. Verfahren nach Anspruch 1, worin das Verfahren kontinuierlich ist.

3. Verfahren nach Anspruch 1 oder 2, worin der makrocyclische Oligoester eine strukturelle Wiederholungseinheit umfasst, die aus der Gruppe ausgewählt ist, die aus Folgenden besteht: Ethylenterephthalat, Propylenterephthalat, 1,3-Propylenterephthalat, 1,4-Butylenterephthalat, 1,4-Cyclohexylendimethylenterephthalat und 1,2-Ethylen-2,6-naphthalendicarboxylat.

4. Verfahren nach Anspruch 1 oder 2, worin der makrocyclische Oligoester die strukturelle Wiederholungseinheit 1,4-Butylenterephthalat umfasst.

5. Verfahren nach einem der vorstehenden Ansprüche, das weiter den Schritt des Zugebens von mindestens einem Zusatzstoff zum makrocyclischen Oligoester umfasst, worin der mindestens eine Zusatzstoff aus der Gruppe ausgewählt ist, die aus Folgenden besteht: einem Farbstoff, einem Pigment, einem magnetischen Stoff, einem Antioxidationsmittel, einem UV-Stabilisator, einem Weichmacher, einem Flammschutzmittel, einem Gleitmittel, einem Formtrennmittel, pyrogenem Silikat, Titandioxid, Calciumcarbonat, geschnittener Faser, Flugasche, Mikroglaskugeln, Mikrohohlkugeln, zertrümmertem Stein, Nanoclay, linearen Polymeren, Monomeren und Katalysator.

6. Verfahren nach Anspruch 5, worin Schritt (b) das Formen einer Mischung umfasst, die den makrocyclischen Oligoester und einen Katalysator umfasst.

7. Verfahren nach einem der vorstehenden Ansprüche, worin der Schritt des Teilkristallisierens des makrocyclischen Oligoesters Scheren, Kühlen oder gleichzeitiges Scheren und Kühlen umfasst.

8. Verfahren nach einem der vorstehenden Ansprüche, worin der Schritt des Teilkristallisierens des makrocyclischen Oligoesters Mischen oder Extrudieren des makrocyclischen Oligoesters umfasst.

9. Verfahren nach einem der vorstehenden Ansprüche, worin der Schritt des Teilkristallisierens des makrocyclischen Oligoesters bei einer Temperatur innerhalb eines Bereichs von etwa 145 °C und etwa 155 °C ausgeführt wird.

10. Verfahren nach einem der vorstehenden Ansprüche, das weiter die folgenden Schritte umfasst:
(i) vor Schritt (a) Bereitstellen einer Lösung, die den makrocyclischen Oligoester und ein Lösungsmittel umfasst; und
(ii) Entfernen des Lösungsmittels.

11. Verfahren nach Anspruch 10, worin der makrocyclische Oligoester einen Lösungsmittelgehalt von weniger als 200 ppm im Anschluss an Schritt (ii) hat.

12. Verfahren nach Anspruch 10, worin der makrocyclische Oligoester einen Lösungsmittelgehalt von weniger als 50 ppm im Anschluss an Schritt (ii) hat.

13. Verfahren nach einem der Ansprüche 10 bis 12, worin Schritt (ii) das Entfernen des Lösungsmittels bei einer erhöhten Temperatur oder bei einem reduzierten Druck oder beidem umfasst.

14. Verfahren nach Anspruch 13, worin Schritt (ii) innerhalb eines Temperaturbereichs von etwa Umgebungstemperatur bis etwa 300 °C ausgeführt wird.

15. Verfahren nach Anspruch 13, worin Schritt (ii) innerhalb eines Temperaturbereichs von etwa 180 °C bis etwa 200 °C ausgeführt wird.

16. Verfahren nach einem der Ansprüche 13 bis 15, worin Schritt (ii) innerhalb eines Druckbereichs von etwa 0,001 Torr [0,13 Pa] bis etwa 10 Torr [1300 Pa] ausgeführt wird.

17. Verfahren nach einem der Ansprüche 13 bis 15, worin Schritt (ii) innerhalb eines Druckbereichs von etwa 1 Torr [130 Pa] bis etwa 10 Torr [1300 Pa] ausgeführt wird.

18. Verfahren nach einem der Ansprüche 13 bis 17, worin Schritt (ii) das Entfernen des Lösungsmittels ohne die Verwendung eines Antisolvents umfasst.

19. Verfahren nach einem der Ansprüche 13, 16, 17 oder 18, worin Schritt (ii) das Entfernen des Lösungsmittels bei einer Temperatur über 180 °C umfasst.

20. Verfahren nach einem der Ansprüche 13, 16, 17 oder 18, worin Schritt (ii) das Entfernen des Lösungsmittels bei einer Temperatur innerhalb eines Bereichs von 120 °C bis 280 °C umfasst.

21. Verfahren nach einem der Ansprüche 13, 16, 17 oder 18, worin Schritt (ii) das Entfernen des Lösungsmittels bei einer Temperatur innerhalb eines Bereichs von 120 °C bis 200 °C umfasst.

22. Verfahren nach einem der Ansprüche 13, 16, 17 oder 18, worin Schritt (ii) das Entfernen des Lösungsmittels bei einer Temperatur innerhalb eines Bereichs von 200 °C bis 280 °C umfasst.

23. Verfahren nach einem der Ansprüche 13 bis 22, worin Schritt (ii) das Entfernen des Lösungsmittels bei einer Temperatur über einem Schmelzpunkt des makrocyclischen Oligoesters umfasst.

24. Verfahren nach einem der Ansprüche 10 bis 23, worin Schritt (ii) und Schritt (b) kombiniert werden.

25. Verfahren nach einem der vorstehenden Ansprüche, worin Schritt (b) das Pelletieren des teilkristallisierten makrocyclischen Oligoesters umfasst.

26. Verfahren nach einem der vorstehenden Ansprüche, worin Schritt (b) das Schneiden des teilkristallisierten makrocyclischen Oligoesters in Wasser umfasst.

27. Verfahren nach einem der vorstehenden Ansprüche, das weiter den Schritt des Sammelns des Produkts von Schritt (b) umfasst.

28. Verfahren nach einem der vorstehenden Ansprüche, worin Schritt (b) das Formen des teilkristallisierten makrocyclischen Oligoesters in Pellets in Wasser umfasst.

29. Verfahren nach Anspruch 28, worin Schritt (b) mit einem Unterwasser-Pelletierer ausgeführt wird.

30. Verfahren nach Anspruch 28 oder 29, das den Schritt des Teilkristallisierens des makrocyclischen Oligoesters vor Schritt (a) durch Kühlen des geschmolzenen makrocyclischen Oligoesters umfasst.

31. Verfahren nach Anspruch 30, worin der geschmolzene makrocyclische Oligoester auf eine Temperatur zwischen etwa 80 °C und etwa 140 °C gekühlt wird.

32. Verfahren nach Anspruch 30 oder 31, worin der teilkristallisierte makrocyclische Oligoester in Schritt (b) eine Viskosität zwischen etwa 3000 cp und etwa 5000 cP hat.

33. Verfahren nach einem der Ansprüche 28 bis 32, das weiter den Schritt des Entfernens des Wassers mit einem Trockner umfasst.

34. Verfahren nach einem der Ansprüche 1 bis 24, worin Schritt (b) das Pastillieren des teilkristallisierten makrocyclischen Oligoesters unter Verwendung eines Tropfengenerators umfasst.

35. Verfahren nach Anspruch 34, worin der teilkristallisierte makrocyclische Oligoester bei einer Temperatur zwischen etwa 150 °C und etwa 200 °C in den Tropfengenerator eintritt.

36. Verfahren nach Anspruch 34 oder 35, worin der teilkristallisierte makrocyclische Oligoester in Schritt (b) eine Viskosität zwischen etwa 500 cP und etwa 1000 cP hat.

37. Verfahren nach einem der Ansprüche 34 bis 36, worin Schritt (b) das Tropfen einer Mischung, die den makrocyclischen Oligoester und einen Katalysator umfasst, auf ein Gewebe zur Bildung eines Prepregs umfasst.

38. Geformter makrocyclischer Oligoester, der durch ein Verfahren nach einem der vorstehenden Ansprüche gebildet wird.

39. Geformter makrocyclischer Oligoester nach Anspruch 38 in Form eines Pellets, einer Pastille, einer Flocke oder eines Prepregs.

40. Geformter makrocyclischer Oligoester nach Anspruch 38 in Form eines Pellets oder einer Pastille.

## Revendications

1. Procédé de mise en forme d'un oligoester macrocyclique, le procédé comprenant les étapes consistant à :
(a) cristalliser partiellement un oligoester macrocyclique comprenant une unité structurale récurrente de formule (I) : 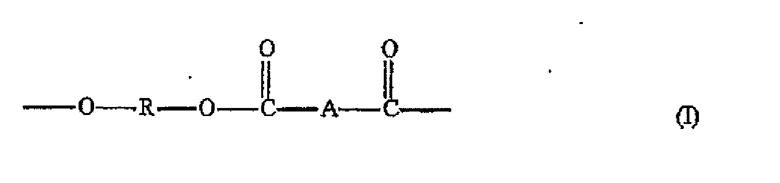 dans laquelle R est un alkylène, un cycloalkylène ou un groupe mono- ou polyoxyalkylène et A est un groupe aromatique ou alicyclique bivalent ; et
(b) mettre en forme l'oligoester macrocyclique partiellement cristallisé en vue de former des granules, des pastilles, des paillettes ou des préimprégnés.

2. Procédé selon la revendication 1, dans lequel le procédé est continu.

3. Procédé selon la revendication 1 ou 2, dans lequel l'oligoester macrocyclique comprend une unité structurale récurrente choisie dans le groupe constitué de l'éthylène téréphtalate, du propylène téréphtalate, du 1,3-propylène téréphtalate, du 1,4-butylène téréphtalate, du 1,4-cyclohexylènediméthylène téréphtalate et du 1,2-éthylène 2,6-naphtalènedicarboxylate.

4. Procédé selon la revendication 1 ou 2, dans lequel l'oligoester macrocyclique comprend l'unité structurale récurrente 1,4-butylène téréphtalate.

5. Procédé selon l'une quelconque des revendications précédentes, comprenant en outre l'étape consistant à ajouter au moins un additif à l'oligoester macrocyclique, où ledit au moins un additif est choisi dans le groupe constitué d'un colorant, d'un pigment, d'une substance magnétique, d'un antioxydant, d'un stabilisateur d'UV, d'un plastifiant, d'un ignifuge, d'un lubrifiant, d'un agent de démoulage, de silicate pyrogéné, de dioxyde de titane, de carbonate de calcium, de fibres coupées, de cendres volantes, de microsphères de verre, de microballons, de pierre écrasée, de nanoargile, de polymères linéaires, de monomères et de catalyseur.

6. Procédé selon la revendication 5, dans lequel l'étape (b) comprend la mise en forme d'un mélange comprenant l'oligoester macrocyclique et un catalyseur.

7. Procédé selon l'une quelconque des revendications précédentes, dans lequel l'étape consistant à cristalliser partiellement l'oligoester macrocyclique comprend le cisaillement, le refroidissement ou le cisaillement et le refroidissement simultanés.

8. Procédé selon l'une quelconque des revendications précédentes, dans lequel l'étape consistant à cristalliser partiellement l'oligoester macrocyclique comprend le mélange ou l'extrusion de l'oligoester macrocyclique.

9. Procédé selon l'une quelconque des revendications précédentes, dans lequel l'étape consistant à cristalliser partiellement l'oligoester macrocyclique est réalisée à une température comprise dans une plage d'environ 145°C et environ 155°C.

10. Procédé selon l'une quelconque des revendications précédentes, comprenant en outre les étapes consistant à :
(i) préalablement à l'étape (a), fournir une solution comprenant l'oligoester macrocyclique et un solvant ; et
(ii) éliminer le solvant.

11. Procédé selon la revendication 10, dans lequel l'oligoester macrocyclique présente une teneur en solvant inférieure à 200 ppm après l'étape (ii).

12. Procédé selon la revendication 10, dans lequel l'oligoester macrocyclique présente une teneur en solvant inférieure à 50 ppm après l'étape (ii).

13. Procédé selon l'une quelconque des revendications 10 à 12, dans lequel l'étape (ii) comprend l'élimination du solvant à une température élevée, ou à une pression réduite, ou les deux.

14. Procédé selon la revendication 13, dans lequel l'étape (ii) est réalisée dans une plage de températures d'environ la température ambiante à environ 300°C.

15. Procédé selon la revendication 13, dans lequel l'étape (ii) est réalisée dans une plage de températures d'environ 180°C à environ 200°C.

16. Procédé selon l'une quelconque des revendications 13 à 15, dans lequel l'étape (ii) est réalisée dans une plage de pressions d'environ 0,001 torr [0,13 Pa] à environ 10 torr [1300 Pa].

17. Procédé selon l'une quelconque des revendications 13 à 15, dans lequel l'étape (ii) est réalisée dans une plage de pressions d'environ 1 torr [130 Pa] à environ 10 torr [1300 Pa].

18. Procédé selon l'une quelconque des revendications 13 à 17, dans lequel l'étape (ii) comprend l'élimination du solvant sans utiliser d'anti-solvant.

19. Procédé selon l'une quelconque des revendications 13, 16, 17 ou 18, dans lequel l'étape (ii) comprend l'élimination du solvant à une température supérieure à 180°C.

20. Procédé selon l'une quelconque des revendications 13,16,17 ou 18, dans lequel l'étape (ii) comprend l'élimination du solvant à une température dans une plage de 120°C à 280°C.

21. Procédé selon l'une quelconque des revendications 13, 16, 17 ou 18, dans lequel l'étape (ii) comprend l'élimination du solvant à une température dans une plage de 120°C à 200°C.

22. Procédé selon l'une quelconque des revendications 13,16,17 ou 18, dans lequel l'étape (ii) comprend l'élimination du solvant à une température dans une plage de 200°C à 280°C.

23. Procédé selon l'une quelconque des revendications 13 à 22, dans lequel l'étape (ii) comprend l'élimination du solvant à une température supérieure à un point de fusion de l'oligoester macrocyclique.

24. Procédé selon l'une quelconque des revendications 10 à 23, dans lequel l'étape (ii) et l'étape (b) sont combinées.

25. Procédé selon l'une quelconque des revendications précédentes, dans lequel l'étape (b) comprend la granulation de l'oligoester macrocyclique partiellement cristallisé.

26. Procédé selon l'une quelconque des revendications précédentes, dans lequel l'étape (b) comprend la coupe de l'oligoester macrocyclique partiellement cristallisé dans de l'eau.

27. Procédé selon l'une quelconque des revendications précédentes, comprenant en outre l'étape consistant à récupérer le produit de l'étape (b).

28. Procédé selon l'une quelconque des revendications précédentes, dans lequel l'étape (b) comprend la mise en forme de l'oligoester macrocyclique partiellement cristallisé en granules dans de l'eau.

29. Procédé selon la revendication 28, dans lequel l'étape (b) est réalisée à l'aide d'un granulateur immergé.

30. Procédé selon la revendication 28 ou 29, comprenant l'étape consistant à cristalliser partiellement l'oligoester macrocyclique avant l'étape (a), en refroidissant l'oligoester macrocyclique fondu.

31. Procédé selon la revendication 30, dans lequel l'oligoester macrocyclique fondu est refroidi à une température comprise entre environ 80°C et environ 140°C.

32. Procédé selon la revendication 30 ou 31, dans lequel l'oligoester macrocyclique partiellement cristallisé dans l'étape (b) présente une viscosité comprise entre environ 3000 cP et environ 5000 cP.

33. Procédé selon l'une quelconque des revendications 28 à 32, comprenant en outre l'étape consistant à éliminer l'eau à l'aide d'un séchoir.

34. Procédé selon l'une quelconque des revendications 1 à 24, dans lequel l'étape (b) comprend le pastillage de l'oligoester macrocyclique partiellement cristallisé au moyen d'un générateur de gouttelettes.

35. Procédé selon la revendication 34, dans lequel l'oligoester macrocyclique partiellement cristallisé entre dans le générateur de gouttelettes à une température comprise entre environ 150°C et environ 200°C.

36. Procédé selon la revendication 34 ou 35, dans lequel l'oligoester macrocyclique partiellement cristallisé dans l'étape (b) présente une viscosité comprise entre environ 500 cP et environ 1000 cP.

37. Procédé selon l'une quelconque des revendications 34 à 36, dans lequel l'étape (b) comprend le fait de laisser tomber un mélange comprenant l'oligoester macrocyclique et un catalyseur sur un tissu pour former un préimprégné.

38. Oligoester macrocyclique mis en forme, formé par un procédé selon l'une quelconque des revendications précédentes.

39. Oligoester macrocyclique mis en forme selon la revendication 38, sous forme d'un granule, d'une pastille, d'une paillette ou d'un préimprégné.

40. Oligoester macrocyclique mis en forme selon la revendication 38, sous forme d'un granule ou d'une pastille.
